# EUROPEAN PATENT SPECIFICATION

(11) **EP 3 003 429 B1**
(45) Date of publication and mention of the grant of the patent: **26.02.2020**
(21) Application number: 14801282.6
(22) Date of filing: 23.05.2014
(51) Int. Cl.: A61M 5/168, A61M 5/172, A61M 5/36

(54) **MULTI-SENSOR INFUSION SYSTEM FOR DETECTING AIR OR AN OCCLUSION IN THE INFUSION SYSTEM**
MULTISENSOR-INFUSIONSSYSTEM ZUR DETEKTION VON LUFT ODER EINER VERSTOPFUNG IN DEM INFUSIONSSYSTEM
SYSTÈME DE PERFUSION À MULTIPLES CAPTEURS POUR DÉTECTER LA PRÉSENCE D'AIR OU D'UNE OCCLUSION DANS LE SYSTÈME DE PERFUSION

(30) Priority: 24.05.2013 US 201361827111 P
(43) Date of publication of application: 13.04.2016
(73) Proprietor: ICU Medical, Inc., San Clemente, CA 92673 (US)
(72) Inventor: ORUKLU, Meriyan, Chicago, Illinois 60657 (US); RUCHTI, Timothy, L., Gurnee, Illinois 60031 (US); KOTNIK, Paul, T., Commerce Township, Michigan 48382 (US); BELKIN, Anatoly, S., Glenview, Illinois 60026 (US); MARKEY, Brian, G., Park Forest, Illinois 60466 (US)
(74) Representative: Invent Horizon IP
(86) International application number: PCT/US2014/039347
(87) International publication number: WO 2014/190264

(56) References cited:
- WO-A1-2009/026420
- WO-A1-2013/028524
- US-A- 5 800 387
- US-A1- 2008 125 701
- US-A1- 2010 185 142
- US-A1- 2011 046 558
- US-A1- 2013 085 689
- US-B1- 6 347 553
- US-B2- 8 177 739

## Description

### FIELD OF THE DISCLOSURE

This disclosure relates to detection systems and methods for detecting air or occlusions in an infusion system.

### BACKGROUND

Existing strategies for detecting air in the line of an infusion device often involve the use of ultrasonic sensors that are physically located on opposite sides of a tubing segment. When fluid is present in the tube, propagation of the acoustic signal is efficient and produces a large electrical signal via the receiver circuit. On the other hand, the presence of air in the tube causes an acoustical open circuit which substantially attenuates the detected signal. In current practice, detection of air in the tubing segment is performed on the basis of a simple (static) air-fluid boundary or threshold that is applied to the sensor voltage signal. When the air sensor signal moves beyond the pre-defined air/fluid threshold, an alarm condition occurs and the IV infusion is paused.

Additionally, in current practice, there exist methods/algorithms that utilize the plunger force sensor readings to detect the presence of air in the plunger chamber. Several Hospira™ pumps involve the use of a cassette with a chamber that is compressed by an actuated plunger to pump fluid at a controlled rate from the drug container to the patient. The measured force during a pumping cycle is directly related to the type of fluid in the chamber. For instance, fluids are relatively incompressible and generate a higher and different force profile than air. Similarly, a combination of fluid and air in the chamber results in a hybrid force profile that is indicative of the mixture percentages.

Both methods described above rely on observations from a single sensor (i.e., air sensor or force sensor). Faulty sensor observations are the major drawback of such single-sensor based systems/algorithms. For instance, for air sensor based algorithms, a variety of situations (e.g., dancing micro air bubbles, stuck fluid droplet at the end-of-bag, etc.) exist which generate false alarms or mask the presence of air in front of the air-sensor leading to false negatives. Similarly, force sensor based algorithms can be fooled by variable distal/proximal pressure during delivery (e.g., kinked tubing due to patient movement). The measured force during a pumping cycle is affected by the pressure applied to both distal and proximal sides of the tubing. For instance, drop in a distal pressure will cause drop in the plunger force readings, which will be perceived as a transition from fluid to air in the chamber by the existing force algorithms and cause a false positive detection of air. Single-sensor based air-in-line detection systems may fail to detect an end-of-bag situation that can result in air in the line, or may incorrectly determine that the fluid in the line is air (i.e., causing nuisance alarms).

Document US 5800387 discloses an infusion system having different types of sensors for indicating air in a delivery line.

A system and method is needed to overcome one or more issues of one or more of the existing infusion systems or methods.

### SUMMARY

An infusion system is disclosed for being operatively connected to a fluid delivery line and to an infusion container containing an infusion fluid. The infusion system includes a pump, a plurality of different types of sensors connected to the pump or the fluid delivery line, at least one processor, and a memory. The plurality of different types of sensors are configured to indicate whether air is in the fluid delivery line. The at least one processor is in electronic communication with the pump and the plurality of different types of sensors. The memory is in electronic communication with the at least one processor. The memory includes programming code for execution by the at least one processor. The programming code is configured to, based on measurements taken by the plurality of different types of sensors, determine the following: (1) whether there is air in the fluid delivery line; (2) whether there is a partial occlusion or a total occlusion in the fluid delivery line; or (3) a percentage of the air present in the fluid delivery line or the probability of the air being in the fluid delivery line.

In another embodiment, a method for infusing an infusion fluid is disclosed. In one step, infusion fluid is pumped through a fluid delivery line of an infusion system. In another step, measurements are taken with a plurality of different types of sensors connected to the infusion system. In an additional step, at least one processor determines, based on the measurements taken by the plurality of the different types of the sensors, the following: (1) whether there is air in the fluid delivery line; (2) whether there is a partial occlusion or a total occlusion in the fluid delivery line; or (3) a percentage of the air present in the fluid delivery line or the probability of the air being in the fluid delivery line.

The scope of the present disclosure is defined solely by the appended claims and is not affected by the statements within this summary.

### BRIEF DESCRIPTION OF THE DRAWINGS

The disclosure can be better understood with reference to the following drawings and description. The components in the figures are not necessarily to scale, emphasis instead being placed upon illustrating the principles of the disclosure.
Figure 1 illustrates a block diagram of one embodiment of an infusion system;
Figure 2 illustrates a flowchart of one embodiment of a method for determining whether air is present in an infusion system;
Figure 3 illustrates a flowchart of another embodiment of a method for determining whether air is in an infusion system;
Figure 4 illustrates a flowchart of still another embodiment of a method for determining whether air is in an infusion system;
Figure 5 illustrates a flowchart of another embodiment of a method for determining whether air is in an infusion system using both plunger force sensor readings and air-in-line sensor readings;
Figure 6 illustrates a flowchart of another embodiment of a method for determining whether air is in an infusion system using both plunger force sensor readings and air-in-line sensor readings;
Figure 7 illustrates a flowchart of another embodiment of a method for determining whether air is in an infusion system using both plunger force sensor readings and air-in-line sensor readings;
Figure 8 illustrates a flowchart of another embodiment of a method for determining whether air is in an infusion system using both plunger force sensor readings and air-in-line sensor readings;
Figure 9 illustrates two related graphs illustrating how the use of a single-sensor based algorithm for detecting the presence of air within an infusion system can lead to a false positive detection of air in the infusion system;
Figure 10 illustrates three related graphs illustrating how the use of a multi-sensor based algorithm for detecting the presence of air within the infusion system tested in Figure 9 eliminates the false positive detection of air in the in the infusion system;
Figure 11 illustrates a flowchart of one embodiment of a method for determining whether air is in an infusion system;
Figure 12 illustrates a flowchart of another embodiment of a method for determining whether air is in an infusion system using plunger force sensor readings and pressure sensor readings;
Figure 13 illustrates two related graphs illustrating how the use of a single-sensor based algorithm for detecting the presence of air within an infusion system can lead to a false positive detection of air in the infusion system;
Figure 14 illustrates three related graphs illustrating how the use of a multi-sensor based algorithm for detecting the presence of air within the infusion system tested in Figure 13 eliminates the false positive detection of air in the in the infusion system;
Figure 15 illustrates a flowchart of an embodiment of a method for determining a probability of air being in an infusion system;
Figure 16 illustrates a flowchart of an embodiment of a method for determining whether a partial or total distal occlusion is present in an infusion system;
Figure 17 illustrates a flowchart of another embodiment of a method for determining whether a partial or total proximal occlusion is present in an infusion system; and
Figure 18 illustrates a flowchart of another embodiment of a method of detecting a partial or total occlusion in an infusion system.

### DETAILED DESCRIPTION

The following detailed description is of the best currently contemplated modes of carrying out the disclosure.

In this disclosure, multi-sensor algorithms that utilize signals from at least two different sensors, such as air, force, and pressure sensors, are utilized. Further, methods are disclosed of combining and qualifying the signals from multi-sensors to improve the robustness and reliability (i.e., true negative and false positive performance) of air detection systems.

The disclosure is a software based solution for detecting the presence of air within a fluid delivery line. The target application is an air-in-line and end-of-bag detection system for IV medication infusion pumps (e.g., Symbiq™, Gemstar™, or Plum™).

In the disclosure signals from multiple-sensors (i.e., acoustic air sensor, force sensor, distal and proximal pressure sensors) are integrated in order to improve the robustness, and the true negative and false positive performance of IV infusion air-in-line detection systems. Disclosed herein are methods of combining and qualifying the signals from multi-sensors to improve the reliability of air detection systems.

In an alternate embodiment, the disclosure can be used to fully characterize the type of fluid-air mixture present in the infusion line by using multiple-sensor signals to determine the percent of air present or the probability of the presence of air. In another alternate embodiment, the disclosure can be used to improve the robustness and reliability of occlusion detection systems by combining and qualifying the signals from multi-sensors.

The following is a summary of some distinguishing elements of this disclosure. An event detection and qualifier algorithm is disclosed which determines the presence of air in the line during delivery on the basis of air sensor and plunger force sensor observations. An event detection and qualifier algorithm is disclosed that determines the presence of air in the line during delivery on the basis of air sensor, plunger force sensor, and distal and proximal pressure sensor observations. An event detection and qualifier algorithm is disclosed that determines the presence of a partial or total distal/proximal occlusion in the fluid delivery line on the basis of plunger force and pressure sensor signals. A multivariate pattern recognition system is disclosed which determines the percent of air present or the probability of the presence of air in the line.

One problem addressed in this disclosure is to integrate signals from multi-sensors in order to improve the robustness, and the true negative and false positive performance of IV infusion air-in-line detection systems. Disclosed herein are methods of combining and qualifying the signals from multi-sensors to improve the reliability of air detection systems.

Another problem addressed in this disclosure is to fully characterize the type of fluid/air mixture present in the infusion line. Disclosed herein are methods that integrate signals from multi-sensors in order to determine the probability or the percent of air present in the line.

Still another problem addressed in this disclosure is the detection of partial and total distal/proximal occlusion in the fluid delivery line. Disclosed herein are methods of combining and qualifying the signals from multi-sensors to improve the robustness and reliability of occlusion detection systems. In current practice, distal/proximal occlusion algorithms are typically based on pressure readings only.

The disclosure improves the air detection capability of existing infusion pump systems that rely on sensors to make a real-time assessment. In doing so, the disclosed methods do not require additional hardware modifications but instead leverage the acquired multi-sensor signals. Additionally, the disclosure does not necessarily replace existing software modules for air detection but adds an additional safety layer.

The disclosure provides a method for improving the robustness of air detection systems by reducing the likelihood of a false positive air detection. This reduces the chances of an interruption of therapy due to a false alarm. The disclosure further provides a means to improve the sensitivity and specificity of air detection by fusing data collected by multiple sensors.

In current practice, air-in-line algorithms are typically based on air sensor signals only and are used to signify the presence of a single bubble, froth, stuck droplet, or cumulative air in the fluid delivery line. Similarly, plunger force algorithms that are based on plunger force signal only, are typically used to signify the presence of air in the plunger chamber. In this disclosure, plunger force algorithms are integrated with air-in-line algorithms to provide a more robust air-in-line detection system with improved true negative and false positive performance.

There is a delay between force and air sensor readings due to the physical location of the two sensors. For instance, for a Symbiq™ pump, the force sensor is located on the plunger and the air sensor is located distal to the plunger, and the fluid volume between the two sensors is approximately 150 µL (or 2 full plunger strokes). The integrated system disclosed herein utilizes both force and air sensor signals to account for such delays.

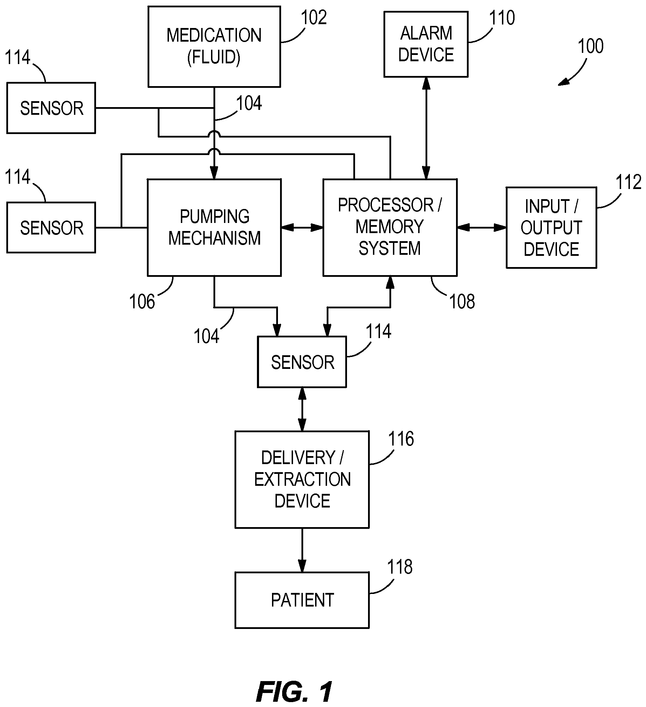

Figure 1 illustrates a block diagram of an infusion system 100 under one embodiment of the disclosure. The infusion system 100 comprises: an infusion container 102; a fluid delivery line 104; a pump device 106; a processing device 108; an alarm device 110 that generates an audio, visual, or other sensory signal or the like to a user; an input/output device 112; a plurality of different types of sensors 114; and a delivery/extraction device 116. The infusion system 100 may comprise an infusion system such as the Plum™, Gemstar™, Symbiq™, or other type of infusion system.

The infusion container 102 comprises a container for delivering an infusion fluid such as IV fluid or a drug to a patient 118. The fluid delivery line 104 comprises one or more tubes, connected between the infusion container 102, the pump device 106, the plurality of different types of sensors 114, and the delivery/extraction device 116, for transporting infusion fluid from the infusion container 102, through the pump device 106, through the plurality of different types of sensors 114, through the delivery/extraction device 116 to the patient 118. The fluid delivery line 104 may also be used to transport blood, delivered to or extracted from the patient 118 using the delivery/extraction device 116, through the plurality of different types of sensors 114 as a result of a pumping action of the pump device 106. The pump device 106 comprises a pump for pumping infusion fluid from the infusion container 102 or for pumping blood to or from the patient 118. The pump device 106 may comprise a plunger based pump, a peristaltic pump, or another type of pump.

The processing device 108 comprises at least one processor for processing information received from the plurality of different types of sensors 114 and for executing one or more algorithms to determine: (1) whether there is air in the fluid delivery line 104; (2) whether there is a partial or total occlusion in the fluid delivery line 104; (3) or a percentage of air present in the fluid delivery line 104 or the probability of the air being in the fluid delivery line 104. The processing device 108 includes or is in electronic communication with a computer readable memory, containing programming code containing the one or more algorithms for execution by the processor, and a clock. The alarm device 110 comprises an alarm, triggered by the processing device 108, for notifying the clinician (also referred to as 'user' herein) of: (1) whether there is air in the fluid delivery line 104; (2) whether there is a partial or total occlusion in the fluid delivery line 104; (3) or a percentage of air present in the fluid delivery line 104 or the probability of the air being in the fluid delivery line 104. The alarm device 110 may be configured to stop the pump device 106 prior to a significant amount of air being delivered through the fluid delivery line 104 and the delivery/extraction device 116 to the patient 118.

The input/output device 112 comprises a device which allows a clinician to input or receive information. The input/output device 112 allows a clinician to input information such as: medication information regarding the infusion fluid being delivered from the infusion container 102; infusion information regarding the infusion of the infusion fluid being delivered from the infusion container 102; distance information regarding the distance(s) between the plurality of different type of sensors; delay information regarding the delay(s) in measurements between the plurality of different types of sensors 114; the selection of settings for the processing device 108 to apply in using the programming code containing the algorithm(s); or other information that is pertinent to the infusion. The medication information regarding the infusion fluid delivered from the infusion container 102 may comprise a formulation of the infusion fluid, a rate of the infusion fluid, a duration of the infusion fluid, a viscosity of the infusion fluid, a therapy of the infusion fluid, or a property of the infusion fluid. The infusion information regarding the infusion fluid delivered from the infusion container 102 may comprise a volume of the infusion fluid in the infusion container or another parameter regarding the infusion of the infusion fluid. The input/output device 112 may allow a clinician to select and/or confirm a user-inputted medication infusion program to be applied by the processing device 108. The input/output device 112 may further output information to the clinician. In other embodiments, any of the information inputted into the input/output device 112 may be pre-installed into the programming code or the processing device 108.

The plurality of different types of sensors 114 may comprise any number, combination, or configuration of pressure sensors, force sensors, air sensors, or other type of sensors. The pressure sensors may comprise one or more proximal or distal pressure sensors for detecting the amount of pressure in the fluid delivery line 104 proximal or distal to the pump device 106. The amount of pressure detected by the one or more pressure sensors is indicative of whether air, fluid, or some combination thereof is present in the fluid delivery line 104. For instance, US 8,403,908 to Jacobson et al. discloses the use of pressure sensors to determine whether air, fluid, or some combination thereof is present in the fluid delivery line 104. The one or more force sensors may comprise one or more force sensors for detecting the amount of force on a plunger of the pump device 106. The amount of force detected by the one or more force sensors is indicative of whether air, fluid, or some combination thereof is present in the fluid delivery line 104. For instance, USSN 13/851,207 filed 27 March 2013 discloses the use of force sensors to determine whether air, fluid, or some combination thereof is present in the fluid delivery line 104. The one or more air sensors may comprise one or more air sensors for detecting whether air, fluid, or a combination thereof is present in the fluid delivery line 104. The strength of the signal that propagates from the one or more air sensors through the fluid delivery line 104 is indicative of whether air, fluid, or some combination thereof is present in the fluid delivery line 104. For instance, US 7,981,082 to Wang et al. discloses the use of air sensors to determine whether air, fluid, or some combination thereof is present in the fluid delivery line 104. In other embodiments, any number, types, combinations, or configurations of sensors 114 may be used to determine whether air, fluid, or some combination thereof is present in the fluid delivery line 104.

The delivery/extraction device 116 comprises a patient vascular access point device for delivering infusion fluid from the infusion container 102 to the patient 118, or for extracting blood from the patient 118. The delivery/extraction device 116 may comprise a needle, a catheter, a cannula, or another type of delivery/extraction device. In other embodiments, the infusion system 100 of Figure 1 may be altered to vary the components, to take away one or more components, or to add one or more components.

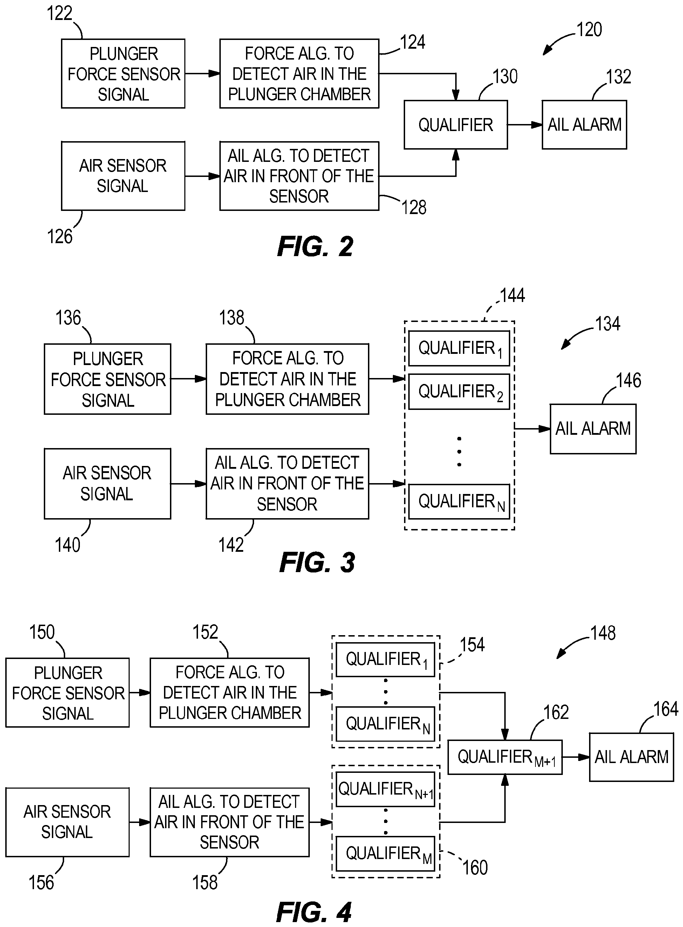

Figure 2 illustrates a flowchart of one embodiment of a method 120 for determining whether air is in an infusion system. The method 120 may utilize the system of Figure 1. In other embodiments, the method 120 may utilize varying systems. In step 122, a force sensor determines how much force is acting upon a plunger or pumping member of a pump. In step 124, a force algorithm is applied using the force sensor measurements of step 122 in order to detect whether air is in a chamber of the pump based on the force sensor measurements. In step 126, an air sensor determines how much of a signal propagates through a fluid delivery line of the infusion system. In step 128, an air-in-line algorithm is applied using the air sensor measurements of step 126 in order to detect whether air is located in the fluid delivery line at the air sensor based on the air sensor measurements. In step 130, a single qualifier algorithm is applied which uses both the results of the application of the force algorithm in step 124 and the results of the application of the air-in-line algorithm of step 128 in order to determine whether air is in the infusion system. The qualifier algorithm of step 130 integrates the decisions of steps 124 and 128 which were based on the measurements of the force sensor and the air sensor and in doing so considers the delay between the force sensor and the air sensor which results due to the distance between them. In such manner, by considering the air results of different types of sensors at different locations a more accurate determination is made as to whether air is contained in the infusion system. In step 132, the alarm device turns on or generates an alarm if step 130 determines that air is in the infusion system. In other embodiments, the method 120 may be altered to vary the order or substance of any of the steps, to delete one or more steps, or to add one or more steps.

Figure 3 illustrates a flowchart of another embodiment of a method 134 for determining whether air is in an infusion system. The method 134 may utilize the system of Figure 1. In other embodiments, the method 134 may utilize varying systems. In step 136, a force sensor determines how much force is acting upon a plunger or pumping member of a pump. In step 138, a force algorithm is applied using the force sensor measurements of step 136 in order to detect whether air is in a chamber of the pump based on the force sensor measurements. In step 140, an air sensor determines how much of a signal propagates through a fluid delivery line of the infusion system. In step 142, an air-in-line algorithm is applied using the air sensor measurements of step 140 in order to detect whether air is located in the fluid delivery line at the air sensor based on the air sensor measurements. In step 144, multiple qualifier algorithms are applied which use both the results of the application of the force algorithm in step 138 and the results of the application of the air-in-line algorithm of step 142 in order to determine whether air is in the infusion system. The multiple qualifier algorithms of step 144 integrate the decisions of steps 138 and 142 which were based on the measurements of the force sensor and the air sensor and in doing so consider the delay between the force sensor and the air sensor which results due to the distance between them. In such manner, by considering the air results of different types of sensors at different locations a more accurate determination is made as to whether air is contained in the infusion system. In step 146, the alarm device generates or turns on an alarm if step 144 determines that air is in the infusion system. In other embodiments, the method 134 may be altered to vary the order or substance of any of the steps, to delete one or more steps, or to add one or more steps.

Figure 4 illustrates a flowchart of still another embodiment of a method 148 for determining whether air is in an infusion system. The method 148 may utilize the system of Figure 1. In other embodiments, the method 148 may utilize varying systems. In step 150, a force sensor determines how much force is acting upon a plunger or pumping member of a pump. In step 152, a force algorithm is applied using the force sensor measurements of step 150 in order to detect whether air is in a chamber of the pump based on the force sensor measurements. In step 154, multiple qualifier algorithms are applied which use the results of the application of the force algorithm in step 152 in order to determine whether air is in the infusion system. In step 156, an air sensor determines how much of a signal propagates through a fluid delivery line of the infusion system. In step 158, an air-in-line algorithm is applied using the air sensor measurements of step 156 in order to detect whether air is located in the fluid delivery line at the air sensor based on the air sensor measurements. In step 160, multiple qualifier algorithms are applied which use the results of the application of the air-in-line algorithm in step 158 in order to determine whether air is in the infusion system. In step 162, a single qualifier algorithm is applied which uses both the results of the multiple qualifier algorithms of step 154 and the results of the multiple qualifier algorithms of step 160 in order to determine whether air is in the infusion system. The qualifier algorithm of step 162 integrates the decisions of steps 154 and 160 which were based on the measurements of the force sensor and the air sensor and in doing so considers the delay between the force sensor and the air sensor which results due to the distance between them. In such manner, by considering the air results of different types of sensors at different locations a more accurate determination is made as to whether air is contained in the infusion system. In step 164, the alarm device turns on or generates an alarm if step 162 determines that air is in the infusion system. In other embodiments, the method 148 may be altered to vary the order or substance of any of the steps, to delete one or more steps, or to add one or more steps.

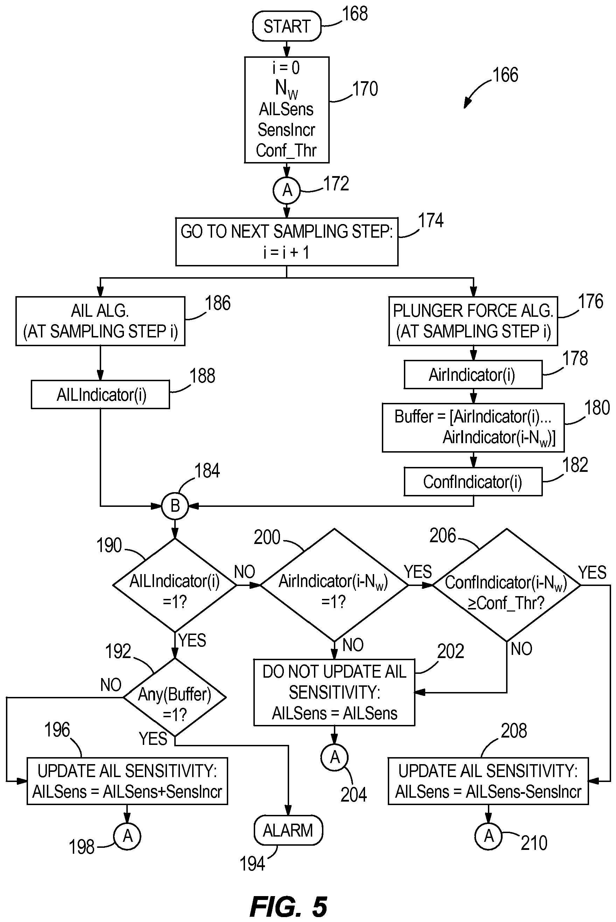

Figure 5 illustrates a flowchart of another embodiment of a method 166 for determining whether air is in an infusion system using both plunger force sensor readings and air-in-line sensor readings. It can be applied to any air-in-line algorithm as long as it outputs an air-in-line indicator at each sampling step indicating whether air was detected in the line by the air-in-line sensor. Similarly, it can be applied to any force algorithm as long as it outputs an air indicator and a confidence indicator at each sampling step indicating whether air was detected in the line by the plunger force sensor and to what confidence level the air indicator based on the plunger force sensor determined that the air was present. The method 166 uses the force algorithm to adjust the sensitivity of the air-in-line algorithm during infusion (i.e. the sensitivity of the air-in-line algorithm is increased when the force algorithm detects air with high confidence and the air-in-line algorithm fails to detect the air; and the sensitivity of the air-in-line algorithm is decreased when the force algorithm does not detect air anywhere in a buffer and the air-in-line algorithm mistakenly detects the air). The method 166 takes into account delays between the air-in-line indicator and the air indicator which result from differences in locations of the air-in-line sensor and the plunger force sensor by utilizing a buffer that stores previous air indicators based on the plunger force sensor measurements. The method 166 may utilize the system of Figure 1. In other embodiments, the method 166 may utilize varying systems.

In step 168, the method starts. The method proceeds from step 168 to step 170. In step 170, the variables are set including setting sampling step i = 0, setting the number Nw of pumping strokes of delay between a plunger force sensor and an air-in-line sensor, setting the air-in-line sensitivity AILSens of the air-in-line sensor to an initial setting, setting the air-in-line sensitivity increment SensIncr of the air-in-line sensor to an initial setting, and setting the percent confidence threshold Conf_Thr to an initial setting. It is noted that throughout this disclosure that sampling step i represents one stroke of the pump of the infusion system. The method proceeds from step 170 through location step 172 to step 174. In step 174, sampling step i is reset to i = i + 1. The method proceeds from step 174 to step 176.

In step 176, a plunger force algorithm is used to determine at sampling step i whether air is detected in a pumping chamber based on measurements of a plunger force sensor. The method proceeds from step 176 to step 178. In step 178, if air is detected in step 176 then an air indicator AirIndicator(i) is set to 1 and if air is not detected in step 176 then the air indicator AirIndicator(i) is set to 0. The method proceeds from step 178 to step 180. In step 180, a Buffer is saved as [AirIndicator(i)...AirIndicator(i-Nw)] saving the 1 or 0 setting made in step 178. For instance, if it takes 2 pumping strokes of the pump for the infusion fluid to travel from the plunger force sensor to the air-in-line sensor, then Nw is set to 2 to accommodate for this delay and the Buffer saves the AirIndicator(i) for the current sample i, the AirIndicator(i-1) for the previous sample i-1, and the AirIndicator(i-2) for two samples before. In step 182, a confidence indicator Conflndicator(i) is set for the current sample i as to the percent confidence in the presence of air being present in the pumping chamber. The method proceeds from step 182 through location step 184 to step 190.

While the method proceeds from step 174 to 176, the method also simultaneously proceeds from step 174 to step 186. In step 186, an air-in-line algorithm is used to determine at sampling step i whether air is detected in a fluid-delivery-line of the infusion system based on measurements from the air-in-line sensor. The method proceeds from step 186 to step 188. In step 188, if air is detected in step 186 then an air-in-line AILIndicator(i) is set to 1 and if air is not detected in step 186 then the air-in-line AILIndicator(i) is set to 0. The method proceeds from step 188 through location step 184 to step 190.

In step 190, a determination is made as to whether the air-in-line indicator AILIndicator(i) equals 1. If a determination is made that the air-in-line indicator AILIndicator(i) does equal 1, then the method proceeds from step 190 to step 192. In step 192, if any of the buffer saved in step 180 is set to 1 (i.e. if any of AirIndicator(i)...AirIndicator(i-Nw) is set to 1), then the method proceeds from step 192 to step 194 and turns on the alarm indicating that air is disposed in the infusion system since both the air-in-line indicator (AILIndicator) and the plunger force indicator (AirIndicator) indicated that air was in the infusion system (i.e. AILIndicator(i) = 1 and one or more of the entries saved in the AirIndicator buffer = 1). When the alarm is turned on in step 194, the infusion system is turned off automatically or manually by the clinician to stop the infusion of the infusion fluid.

In step 192, if any of the buffer saved in step 180 is not set to 1 (i.e. if any of AirIndicator(i)...AirIndicator(i-Nw) is not set to 1), then the method proceeds from step 192 to step 196. In step 196, the air-in-line sensitivity AILSens is updated to decrease the sensitivity using the equation AILSens = AILSens + SensIncr. The air-in-line sensitivity is decreased in step 196 because air was detected by the air-in-line indicator AILIndicator (i.e. AILIndicator(i) = 1) but air was not detected by the plunger force indicator AIRIndicator (i.e. none of the entries in the AirIndicator buffer = 1) which demonstrates that the air-in-line indicator AILIndicator caused a false positive. To increase the robustness of the air-detection system in step 196 the sensitivity of the air-in-line indicator is decreased to reduce the occurrence of false positives. It is noted that to decrease the air-in-line sensitivity in step 196 the AILSens is actually increased because the larger the AILSens is the less sensitive the algorithm will be causing it to only detect larger air-slugs. The method proceeds from step 196 through location step 198 though location step 172 to step 174 and repeats the process steps.

In step 190, if the air-in-line indicator AILIndicator(i) is not set to 1 the method proceeds from step 190 to step 200. In step 200, a determination is made whether AirIndicator(i-Nw) is set to 1 (i.e. whether the plunger force indicator Nw cycles ahead of the air-in-line detector determined that air was in the infusion system to accommodate for the delay between the plunger force sensor and the air-in-line sensor). If the determination is made in step 200 that the AirIndicator(i-Nw) is not set to 1 the method proceeds from step 200 to step 202. In step 202, the AIL Sensitivity is not updated (i.e. AILSens remains equal to AILSens since neither the air-in-line indicator (AILIndicator) nor the plunger force indicator (AirIndicator) indicated that air was in the infusion system). The method proceeds from step 202 through location step 204 through location step 172 to step 174 and repeats the process steps.

If the determination is made in step 200 that the AirIndicator(i-Nw) is set to 1 the method proceeds from step 200 to step 206. In step 206, a determination is made whether the confidence indicator Conflndicator(i-Nw) of the force algorithm (indicating the confidence level that air has been detected in the infusion system by the plunger force sensor by applying the force algorithm Nw cycles ahead of the air-in-line sensor) is greater than or equal to the confidence threshold (Conf_Thr). If the determination is made in step 206 that the confidence indicator of the force algorithm is not greater than or equal to the confidence threshold, the method proceeds from step 206 to step 202. In step 202, the air-in-line sensitivity AILSens is not updated (i.e. AILSens remains equal to AILSens since the air-in-line indicator (AILIndicator) did not indicate that air was in the infusion system and the plunger force indicator (AirIndicator) only indicated with low confidence that air was in the infusion system). The method proceeds from step 202 through location step 204 through location step 172 to step 174 and repeats the process steps.

If the determination is made in step 206 that the confidence indicator Conflndicator(i-Nw) of the force algorithm is greater than or equal to the confidence threshold Conf_Thr, the method proceeds from step 206 to step 208. In step 208, the air-in-line sensitivity is updated to increase the sensitivity using the equation AILSens = AILSens - SensIncr. The air-in-line sensitivity is increased in step 208 because air was not detected by the air-in-line indicator AILIndicator (i.e. AILIndicator(i) was not set to 1) but air was detected by the plunger force indicator AIRIndicator with a high confidence level (AirIndicator(i-Nw) was set to 1 and the Conflndicator(i-Nw) was greater than or equal to Conf_Thr) which demonstrates that the air-in-line indicator AILIndicator was not sensitive enough and caused a missed air-in-line AILIndicator detection of air. To increase the robustness of the air-detection system in step 208 the sensitivity of the air-in-line indicator is increased to reduce the occurrence of missed positive detections of air in the infusion system. It is noted that to increase the air-in-line sensitivity in step 208 the AILSens is actually decreased because the smaller the AILSens is the more sensitive the algorithm will be causing it to detect smaller air-slugs. The method proceeds from step 208 through location step 210 through location step 172 to step 174 and repeats the process steps. In other embodiments, the method 166 may be altered to vary the order or substance of any of the steps, to delete one or more steps, or to add one or more steps.

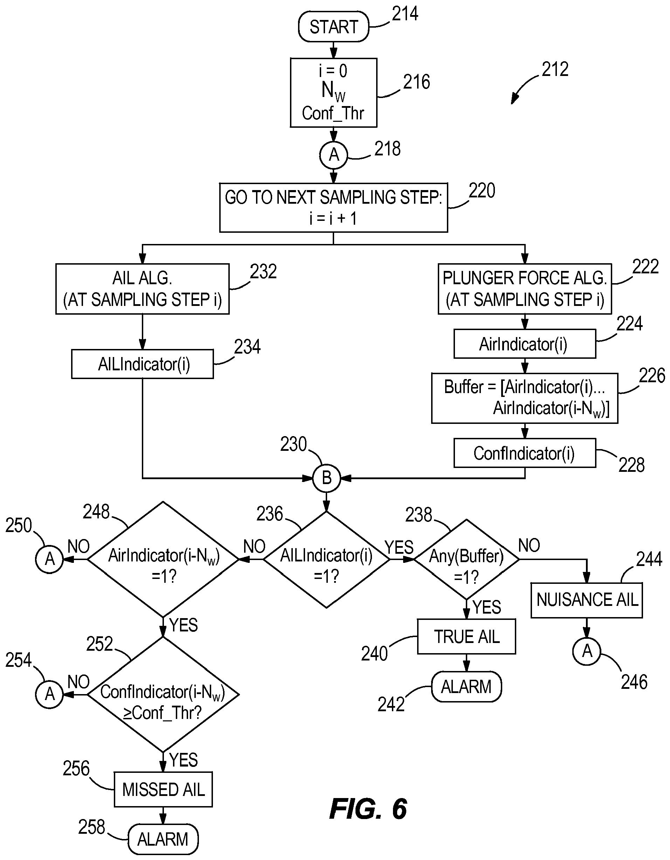

Figure 6 illustrates a flowchart of another embodiment of a method 212 for determining whether air is in an infusion system using both plunger force sensor readings and air-in-line sensor readings. It can be applied to any air-in-line algorithm as long as it outputs an air-in-line indicator at each sampling step indicating whether air was detected in the line by the air-in-line sensor. Similarly, it can be applied to any force algorithm as long as it outputs an air indicator and a confidence indicator at each sampling step indicating whether air was detected in the line by the plunger force sensor and to what confidence level the air indicator based on the plunger force sensor determined that the air was present. The method 212 uses the force algorithm to declare true, missed, or nuisance/false air-in-line alarms. The method 212 takes into account delays between the air-in-line indicator and the air indicator which result from differences in locations of the air-in-line sensor and the plunger force sensor by utilizing a buffer that stores previous air indicators based on the plunger force sensor measurements. The method 212 may utilize the system of Figure 1. In other embodiments, the method 212 may utilize varying systems.

In step 214, the method starts. The method proceeds from step 214 to step 216. In step 216, the variables are set including setting sampling step i = 0, setting the number Nw of pumping strokes of delay between the plunger force sensor and an air-in-line sensor, and setting the percent confidence threshold Conf_Thr to an initial setting. The method proceeds from step 216 through location step 218 to step 220. In step 220, sampling step i is reset to i = i + 1. The method proceeds from step 220 to step 222.

In step 222, a plunger force algorithm is used to determine at sampling step i whether air is detected in a pumping chamber based on measurements of a plunger force sensor. The method proceeds from step 222 to step 224. In step 224, if air is detected in step 222 then an air indicator AirIndicator(i) is set to 1 and if air is not detected in step 222 then the air indicator AirIndicator(i) is set to 0. The method proceeds from step 224 to step 226. In step 226, a Buffer is saved as [AirIndicator(i)...AirIndicator(i-Nw)] saving the 1 or 0 setting made in step 224. For instance, if it takes 2 pumping strokes of the pump for the infusion fluid to travel from the plunger force sensor to the air-in-line sensor, then Nw is set to 2 to accommodate for this delay and the Buffer saves the AirIndicator(i) for the current sample i, the AirIndicator(i-1) for the previous sample i-1, and the AirIndicator(i-2) for two samples before. In step 228, a confidence indicator Conflndicator(i) is set for the current sample i as to the percent confidence in the presence of air being present in the pumping chamber. The method proceeds from step 228 through location step 230 to step 236.

While the method proceeds from step 220 to 222, the method also simultaneously proceeds from step 220 to step 232. In step 232, an air-in-line algorithm is used to determine at sampling step i whether air is detected in a fluid-delivery-line of the infusion system based on measurements from the air-in-line sensor. The method proceeds from step 232 to step 234. In step 234, if air is detected in step 232 then an air-in-line AILIndicator(i) is set to 1 and if air is not detected in step 232 then the air-in-line AILIndicator(i) is set to 0. The method proceeds from step 234 through location step 230 to step 236.

In step 236, a determination is made as to whether the air-in-line indicator AILIndicator(i) equals 1. If it is determined in step 236 that the air-in-line indicator AILIndicator(i) equals 1, the method proceeds from step 236 to step 238. In step 238, if any of the buffer saved in step 226 is set to 1 (i.e. if any of AirIndicator(i)...AirIndicator(i-Nw) is set to 1), the method proceeds from step 238 to step 240 and determines that there is air in the infusion system. The method proceeds from step 240 to step 242 and turns on the alarm indicating that air is disposed in the infusion system since both the air-in-line indicator (AILIndicator) and the plunger force indicator (AirIndicator) indicated that air was in the infusion system (i.e. AILIndicator(i) = 1 and one or more of the entries saved in the AirIndicator buffer = 1). When the alarm is turned on in step 242, the infusion system is turned off automatically or manually by the clinician to stop the infusion of the infusion fluid.

In step 238, if any of the buffer saved in step 226 is not set to 1 (i.e. if any of AirIndicator(i)...AirIndicator(i-Nw) is not set to 1), the method proceeds from step 238 to step 244. In step 244, a determination is made that the air-in-line indicator AILIndicator resulted in a nuisance air-in-line determination since although the air-in-line indicator indicated that air was present no AirIndicator in the buffer indicated that air was present. The method proceeds from step 244 through location step 246 through location step 218 to step 220 and repeats the process steps.

If it is determined in step 236 that the air-in-line indicator AILIndicator(i) does not equal 1, the method proceeds from step 236 to step 248. In step 248, a determination is made whether AirIndicator(i-Nw) is set to 1 (i.e. whether the plunger force indicator Nw cycles ahead of the air-in-line detector determined that air was in the infusion system to accommodate for the delay between the plunger force sensor and the air-in-line sensor). If the determination is made in step 248 that the AirIndicator(i-Nw) is not set to 1 the method proceeds from step 248 through location step 250 through location step 218 to step 220 and repeats the process steps (since neither the air-in-line indicator AILIndicator nor the air indicator AirIndicator indicated that air was in the infusion system).

If the determination is made in step 248 that the AirIndicator(i-Nw) is set to 1 the method proceeds from step 248 to step 252. In step 252, a determination is made whether the confidence indicator Conflndicator(i-Nw) of the force algorithm (indicating the confidence level that air has been detected in the infusion system by the plunger force sensor by applying the force algorithm Nw cycles ahead of the air-in-line sensor) is greater than or equal to the confidence threshold (Conf_Thr). If the determination is made in step 252 that the confidence indicator of the force algorithm is not greater than or equal to the confidence threshold, the method proceeds from step 252 through location step 254 through location step 218 to step 220 and repeats the process steps (since the air-in-line indicator AILIndicator did not indicate that air was in the infusion system and the air indicator AirIndicator did not confidently predict that air was in the infusion system).

If the determination is made in step 252 that the confidence indicator of the force algorithm is greater than or equal to the confidence threshold, the method proceeds from step 252 to step 256. In step 256, a determination is made that the air-in-line indicator AILIndicator wrongly determined that air was not present in the infusion system (since the air-in-line indicator AILIndicator did not indicate that air was in the infusion system but the air indicator AirIndicator confidently determined that air was in the infusion system). The method proceeds from step 256 to step 258. In step 258, the alarm is turned on indicating that air is disposed in the infusion system. When the alarm is turned on in step 258, the infusion system is turned off automatically or manually by the clinician to stop the infusion of the infusion fluid. In other embodiments, the method 212 may be altered to vary the order or substance of any of the steps, to delete one or more steps, or to add one or more steps.

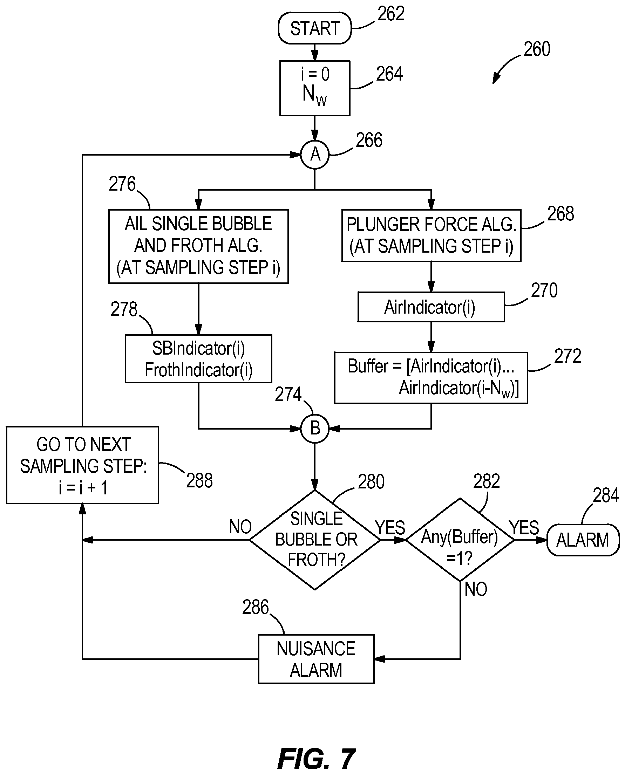

Figure 7 illustrates a flowchart of another embodiment of a method 260 for determining whether air is in an infusion system using both plunger force sensor readings and air-in-line sensor readings. It can be applied to any air-in-line single bubble algorithm or froth algorithm as long as it outputs an air-in-line single bubble indicator and a froth indicator at each sampling step indicating whether a single bubble or froth was detected in the line by the air-in-line sensor. Similarly, it can be applied to any force algorithm as long as it outputs an air indicator at each sampling step indicating whether air was detected in the line by the plunger force sensor. The method 260 uses the force algorithm to declare true air alarms, or nuisance/false air-in-line single bubble or froth alarms. The method 260 takes into account delays between the air-in-line indicator and the air indicator which result from differences in locations of the air-in-line sensor and the plunger force sensor by utilizing a buffer that stores previous air indicators based on the plunger force sensor measurements. The method 260 may utilize the system of Figure 1. In other embodiments, the method 260 may utilize varying systems.

In step 262, the method starts. The method proceeds from step 262 to step 264. In step 264, the variables are set including setting sampling step i = 1 and setting the number Nw of pumping strokes of delay between a plunger force sensor and an air-in-line sensor. The method proceeds from step 264 through location step 266 to step 268. In step 268, a plunger force algorithm is used to determine at sampling step i whether air is detected in a pumping chamber based on measurements of a plunger force sensor. The method proceeds from step 268 to step 270. In step 270, if air is detected in step 268 then an air indicator AirIndicator(i) is set to 1 and if air is not detected in step 268 then the air indicator AirIndicator(i) is set to 0. The method proceeds from step 270 to step 272. In step 272, a Buffer is saved as [AirIndicator(i)...AirIndicator(i-Nw)] saving the 1 or 0 setting made in step 270. For instance, if it takes 2 pumping strokes of the pump for the infusion fluid to travel from the plunger force sensor to the air-in-line sensor, then Nw is set to 2 to accommodate for this delay and the Buffer saves the AirIndicator(i) for the current sample i, the AirIndicator(i-1) for the previous sample i-1, and the AirIndicator(i-2) for two samples before. The method proceeds from step 272 through location step 274 to step 280.

While the method proceeds from step 266 to 268, the method also simultaneously proceeds from step 266 to step 276. In step 276, an air-in-line single bubble algorithm and an air-in-line froth algorithm is used to determine at sampling step i whether a single bubble or forth is detected in a fluid-delivery-line of the infusion system based on measurements from the air-in-line sensor. The method proceeds from step 276 to step 278. In step 278, if a single bubble is detected in step 276 then a single bubble indicator SBIndicator(i) is set to 1 and if the single bubble is not detected in step 276 then the SBIndicator(i) is set to 0. Similarly, in step 278, if froth is detected in step 276 then a froth indicator FrothIndicator(i) is set to 1 and if the froth is not detected in step 276 then the froth indicator FrothIndicator(i) is set to 0. The method proceeds from step 278 through location step 274 to step 280.

In step 280, a determination is made as to whether either the single bubble indicator SBIndicator(i) is set to 1 or the froth indicator FrothIndicator(i) is set to 1. If step 280 determines that either the single bubble indicator SBIndicator(i) is set to 1 or the froth indicator FrothIndicator(i) is set to 1, then the method proceeds from step 280 to step 282. In step 282, a determination is made as to whether any of the buffer saved in step 272 is set to 1 (i.e. if any of AirIndicator(i)...AirIndicator(i-Nw) is set to 1). If step 282 determines that any of the buffer saved in step 272 is set to 1, then the method proceeds from step 282 to step 284 and turns on the alarm indicating that air is disposed in the infusion system since the plunger force indicator (AirIndicator) indicated that air was in the infusion system and either the single bubble indicator (SBIndicator) or the froth indicator (FrothIndicator) indicated that a single bubble or froth was in the infusion system. When the alarm was turned on in step 284, the infusion system is turned off automatically or manually by the clinician to stop the infusion of the infusion fluid.

If step 282 determines that none of the buffer saved in step 272 is set to 1, then the method proceeds from step 282 to step 286. In step 286, a nuisance alarm is turned on because the plunger force indicator AirIndicator found that no air was in the infusion system but the single bubble indicator SBIndicator or the froth indicator FrothIndicator detected that a single bubble or froth was present in the infusion system. The method proceeds from step 286 to step 288. In step 288, sampling step i is incremented to i = i + 1. The method proceeds from step 288 through location step 266 to steps 268 and 276 and repeats the process steps.

If step 280 determines that neither the single bubble indicator SBIndicator(i) is set to 1 nor the froth indicator FrothIndicator(i) is set to 1, then the method proceeds from step 280 to step 288. In step 288, sampling step i is incremented to i = i + 1. The method proceeds from step 288 through location step 266 to steps 268 and 276 and repeats the process steps. In another embodiment, if step 280 determines that neither the single bubble indicator SBIndicator(i) is set to 1 nor the froth indicator FrothIndicator(i) is set to 1, then the method can determine whether the plunger force indicator AirIndicator found that air was in the infusion system and if it did then a missed alarm can be turned on. In other embodiments, the method 260 may be altered to vary the order or substance of any of the steps, to delete one or more steps, or to add one or more steps.

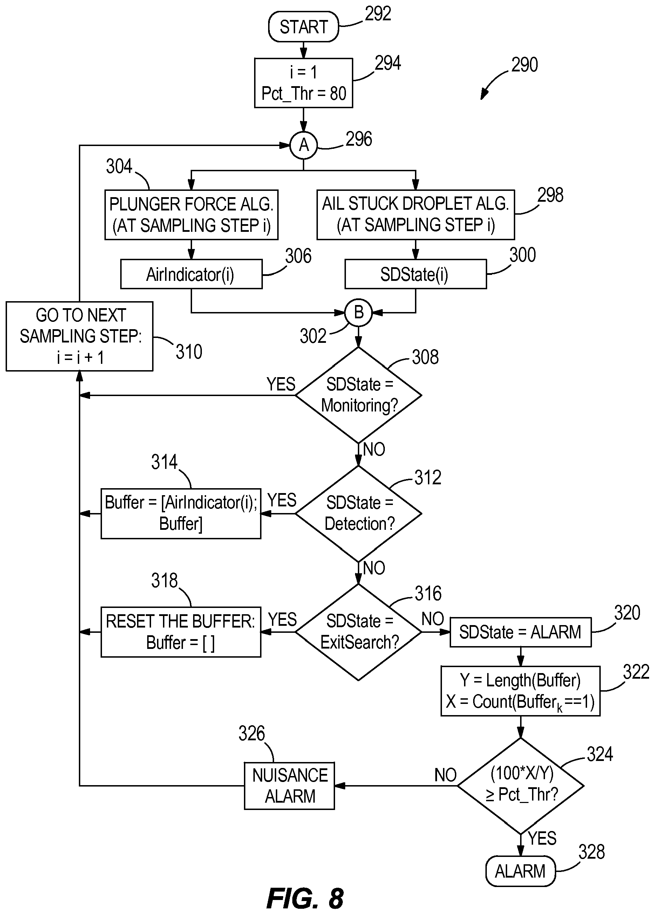

Figure 8 illustrates a flowchart of another embodiment of a method 290 for determining whether air is in an infusion system using both plunger force sensor readings and air-in-line sensor readings. It can be applied to any force algorithm as long as it outputs an air indicator at each sampling step indicating whether air was detected in the line by the plunger force sensor. It can be applied to air-in-line stuck droplet algorithms which determine at each sampling step or pumping stroke whether it is in a monitoring state, a detection state, an exit state, or an alarm state. The method 290 uses the force algorithm and the various states of the air-in-line stuck droplet algorithm to declare true air alarms, or nuisance/false air alarms. The method 290 may utilize the system of Figure 1. In other embodiments, the method 290 may utilize varying systems.

In step 292, the method starts. The method proceeds from step 292 to step 294. In step 294, the variables are set including setting sampling step i = 1, and setting the percent threshold Pct_Thr = 80. The method proceeds from step 294 through location step 296 to step 298. In step 298, an air-in-line stuck droplet algorithm is used to determine at sampling step i whether a stuck droplet is detected in the infusion system based on measurements of an air-in-line sensor. The method proceeds from step 298 to step 300. In step 300, the stuck droplet state at sampling step i SDState(i) is determined as being either in a monitoring state, in a detection state, in an exit search state, or in an alarm state. The method proceeds from step 300 through location step 302 to step 308.

While the method proceeds from location step 296 to step 298, the method also simultaneously proceeds from location step 296 to step 304. In step 304, a plunger force algorithm is used to determine at sampling step i whether air is detected in a pumping chamber based on measurements of a plunger force sensor. The method proceeds from step 304 to step 306. In step 306, if air is detected in step 304 then an air indicator AirIndicator(i) is set to 1 and if air is not detected in step 304 then the air indicator AirIndicator(i) is set to 0. The method proceeds from step 306 through location step 302 to step 308.

In step 308, a determination is made based on the determination of step 300 whether the stuck droplet state at sampling step i SDState(i) is in a monitoring state. In the monitoring state, the algorithm will search for a stuck droplet pattern. If in step 308 the determination is made that the stuck droplet state at sampling step i SDState(i) is in a monitoring state, then the method proceeds to step 310. In step 310, the sampling step i is set to i = i + 1 in order to advance to the next sampling step in order to monitor the next sample (i.e. the next pumping stroke). The method proceeds from step 310 to location step 296 and repeats the process steps.

If in step 308 the determination is made that the stuck droplet state at sampling step i SDState(i) is not in a monitoring state then the method proceeds to step 312. In step 312, a determination is made based on the determination of step 300 whether the stuck droplet state at sampling step i SDState(i) is in a detection state. In the detection state, the algorithm detects a possible stuck droplet pattern and has to decide if it is a match or not. If in step 312 the determination is made that the stuck droplet state at sampling step i SDState(i) is in a detection state, then the method proceeds to step 314. In step 314, a Buffer is saved as [AirIndicator(i)...] saving the AirIndicator(i) determination made in step 306 for the current sample i as being 1 or 0. The Buffer will continue to save all AirIndicator(i) determinations made in step 306 for all samples i until the Buffer is reset. It is noted that the values in the Buffer will later be used to determine in the alarm state whether the air-in-line algorithm decision as to whether air is in the infusion system is a nuisance or a true alarm. The method then proceeds from step 314 to step 310. In step 310, the sampling step i is set to i = i + 1 in order to advance to the next sampling step in order to monitor the next sample (i.e. the next pumping stroke). The method proceeds from step 310 to location step 296 and repeats the process steps.

If in step 312 the determination is made that the stuck droplet state at sampling step i SDState(i) is not in a detection state, then the method proceeds to step 316. In step 316, a determination is made based on the determination of step 300 whether the stuck droplet state at sampling step i SDState(i) is in an exit search state. In the exit search state, the algorithm decides that the suspected stuck droplet pattern is not a match and therefore is not a stuck droplet. If in step 316 the determination is made that the stuck droplet state at sampling step i SDState(i) is in an exit search state, then the method proceeds to step 318. In step 318, the Buffer saved in 314 is reset/cleared so that all previously stored values are deleted. The method proceeds from step 318 to step 310. In step 310, the sampling step i is set to i = i + 1 in order to advance to the next sampling step in order to monitor the next sample (i.e. the next pumping stroke). The method proceeds from step 310 to location step 296 and repeats the process steps.

If in step 316 the determination is made that the stuck droplet state at sampling step i SDState(i) is not in an exit search state, then the method proceeds to step 320. In step 320, a determination is made based on the determination of step 300 that the stuck droplet state at sampling step i SDState(i) is in an alarm state. The method proceeds from step 320 to step 322. In step 322, an X value is saved and a Y value is saved. The X value comprises the number of 1 values saved in the Buffer of step 314. The Y value comprises the overall number of values saved in the Buffer of step 314. For instance, if the Buffer of step 314 is saved as [1 0 0 1 0] then X = 2 because there are two 1 values saved and Y = 5 because there are five overall 1's and 0's saved. The method proceeds from step 322 to step 324. In step 324, a determination is made as to whether 100 multiplied by X/Y is greater than or equal to the percent threshold Pct_Thr of 80 set in step 294 (whether 100 ^{∗} X/Y is greater than or equal to Pct_Thr). If in step 324 the determination is made that 100 multiplied by X/Y is not greater than or equal to the percent threshold Pct_Thr of 80 set in step 294 then the method proceeds to step 326. For instance, if X = 2 and Y = 5 then 100 ^{∗} 2/5 = 40 which is not greater than or equal to Pct_Thr of 80 so the method would proceed to step 326. In step 326, a nuisance alarm is turned on and the infusion is not stopped because less than the threshold number of the buffer determinations, made by the plunger force algorithm, determined that air was in the infusion system leading to the determination that the stuck droplet alarm state set in step 320 was a nuisance alarm. The method proceeds from step 326 to step 310. In step 310, the sampling step i is set to i = i + 1 in order to advance to the next sampling step in order to monitor the next sample (i.e. the next pumping stroke). The method proceeds from step 310 to location step 296 and repeats the process steps.

If in step 324 the determination is made that 100 multiplied by X/Y is greater than or equal to the percent threshold Pct_Thr of 80 set in step 294 then the method proceeds to step 328. For instance, if the Buffer set in step 314 is [1 1 10 1] then X = 4 and Y = 5 and 100 ^{∗} 4/5 = 80 which is greater than or equal to Pct_Thr of 80 so the method would proceed to step 328. In step 328, an alarm is turned on indicating that air is contained in the infusion system because greater than or equal to the threshold number of the buffer determinations, made by the plunger force algorithm, determined that air was in the infusion system leading to the determination that the stuck droplet alarm state set in step 320 was a true air alarm. When the alarm is turned on in step 328, the infusion system is turned off automatically or manually by the clinician to stop the infusion of the infusion fluid. In other embodiments, the method 290 may be altered to vary the order or substance of any of the steps, to delete one or more steps, or to add one or more steps.

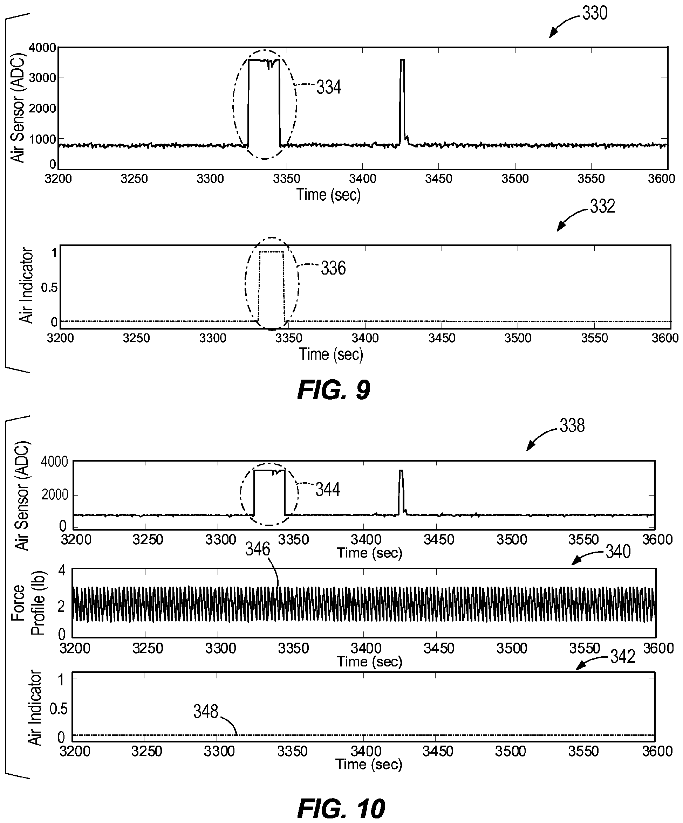

Figure 9 illustrates two related graphs 330 and 332 illustrating how the use of a single-sensor based algorithm for detecting the presence of air within an infusion system can lead to a false positive detection of air in the infusion system. The graphs 330 and 332 were taken from an infusion system which did not contain a significant amount of air to warrant stopping the infusion system. The X-axis of graph 330 represents time in seconds during the infusion of infusion fluid delivered by the infusion system. The Y-axis of graph 330 represents air sensor readings of the single air-in-line sensor in analog-to-digital counts (ADC) for the infusion system during the infusion of the infusion fluid. The portion 334 of the plotted air-sensor readings shows that the air-sensor readings substantially increase and then decrease around 3,325 seconds to 3,350 seconds.

Similarly, the X-axis of graph 332 represents the corresponding time in seconds during the infusion of infusion fluid delivered by the infusion system. The Y-axis of graph 332 represents air indicator determinations made based on the air-in-line sensor readings of the corresponding graph 330. The portion 336 of the plotted air indicator readings shows that the air indicator readings substantially increase and then decrease around 3,325 seconds to 3,350 seconds based on the air-sensor readings of the corresponding graph 330. This portion 336 would result in a false positive detection of air when using a typical single-sensor based air-in-line algorithm. This false positive is caused by dancing micro bubbles of air in the infusion system. This is problematic as the infusion system would be shut down due to this false positive creating an improper delay in therapy to the patient.

Figure 10 illustrates three related graphs 338, 340, and 342 illustrating how the use of a multi-sensor based algorithm for detecting the presence of air within the infusion system tested in Figure 9 eliminates the false positive detection of air in the in the infusion system. The graphs 338, 340, and 342 were taken from the infusion system tested in Figure 9 which did not contain a significant amount of air to warrant stopping the infusion system. The X-axis of graph 338 represents time in seconds during the infusion of infusion fluid delivered by the infusion system. The Y-axis of graph 338 represents air sensor readings of an air-in-line sensor in analog-to-digital counts (ADC) for the infusion system during the infusion of the infusion fluid. The portion 344 of the plotted air-sensor readings shows that the air-sensor readings substantially increase and then decrease around 3,325 seconds to 3,350 seconds.

Similarly, the X-axis of graph 340 represents the corresponding time in seconds during the infusion of infusion fluid delivered by the infusion system. The Y-axis of graph 340 represents force profile readings in pounds taken by a plunger force sensor connected to the infusion system. The plotted portion 346 of the force profile readings shows that the plot is substantially uniform during the entire time plotted from 3,200 seconds to 3,600 seconds which does not indicate that air is in the infusion system.

Similarly, the X-axis of graph 342 represents the corresponding time in seconds during the infusion of infusion fluid delivered by the infusion system. The Y-axis of graph 342 represents air indicator determinations made by integrating the air-sensor readings and the force profile readings of the corresponding graphs 338 and 340. The plotted portion 348 of the air indicator readings shows that the air indicator readings stayed at 0 during the entire time plotted from 3,200 seconds to 3,600 seconds based on the integrated air-sensor readings and force profile readings of the corresponding graphs 338 and 340. As a result, the use of multiple different types of sensors to monitor the infusion system has eliminated the false positive detection of air which occurred when the same infusion system was tested using only a single type of sensor. This improves accuracy and avoids unnecessary shut-downs of the infusion system.

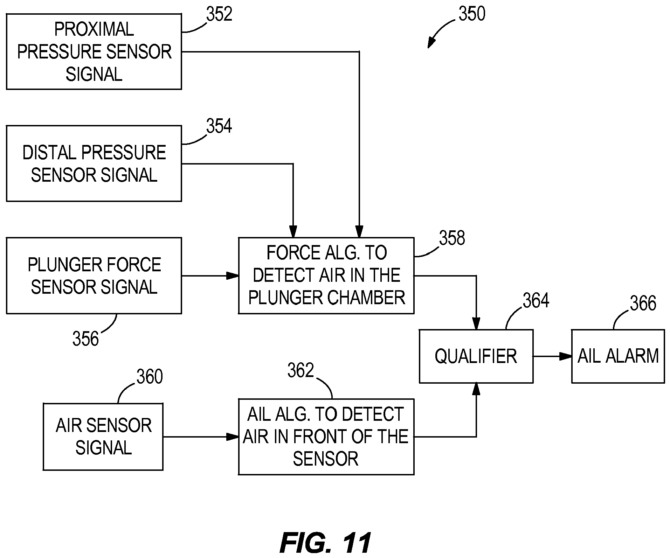

Figure 11 illustrates a flowchart of one embodiment of a method 350 for determining whether air is in an infusion system. The method 350 may utilize the system of Figure 1. In other embodiments, the method 350 may utilize varying systems. In step 352, a proximal pressure sensor determines the amount of pressure acting proximally on the infusion system. In step 354, a distal pressure sensor determines the amount of pressure acting distally on the infusion system. In step 356, a force sensor determines how much force is acting upon a plunger or pumping member of a pump. In step 358, a force algorithm is applied by integrating the proximal pressure measurements, the distal pressure measurements, and the force sensor measurements of steps 352, 354, and 356 in order to detect whether air is in a chamber of the pump. The force algorithm of step 358 integrates the readings of steps 352, 354, and 356 which were based on the measurements of the proximal pressure sensor, the distal pressure sensor, and the force sensor and in doing so considers the delays between the proximal force sensor, the distal pressure sensor, and the force sensor which results due to the distances between them.

In step 360, an air sensor determines how much of a signal propagates through a fluid delivery line of the infusion system. In step 362, an air-in-line algorithm is applied using the air sensor measurements of step 360 in order to detect whether air is located in the fluid delivery line at the air sensor based on the air sensor measurements. In step 364, a single qualifier algorithm is applied which uses both the results of the application of the force algorithm in step 358 and the results of the application of the air-in-line algorithm of step 362 in order to determine whether air is in the infusion system. The qualifier algorithm of step 364 integrates the decisions of steps 358 and 362 which were based on the measurements of the proximal pressure sensor, the distal pressure sensor, the force sensor, and the air sensor and in doing so considers the delays between the proximal force sensor, the distal pressure sensor, the force sensor, and the air sensor which results due to the distances between them.

In such manner, by considering the air results of different types of sensors at different locations a more accurate determination is made as to whether air is contained in the infusion system. This avoids false positives or nuisance alarms caused by a reading by one sensor at one location which is either inaccurate or caused by an issue such as bouncing air bubbles, a stuck droplet, or froth in the infusion system which otherwise would lead to an inaccurate determination as to the presence of air in the infusion system. In step 366, the alarm device turns on an alarm if step 364 determines that air is in the infusion system. In other embodiments, the method 350 may be altered to vary the order or substance of any of the steps, to delete one or more steps, or to add one or more steps.

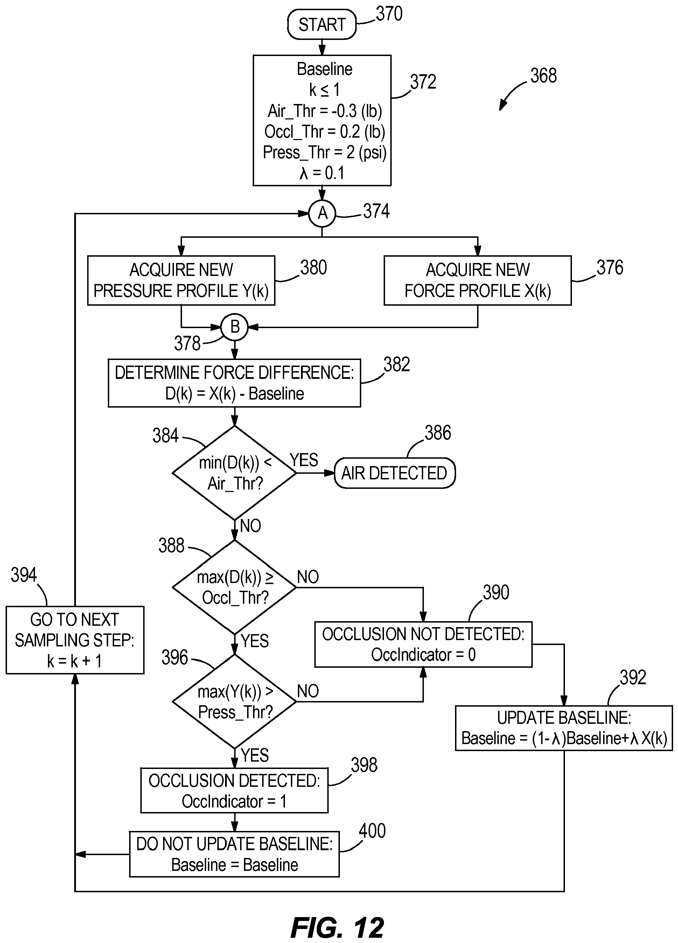

Figure 12 illustrates a flowchart of another embodiment of a method 368 for determining whether air is in an infusion system using plunger force sensor readings and pressure sensor readings. It can be applied as long as a plunger force profile and a pressure profile is taken at each sampling step. The method 368 uses the plunger force profile to determine whether there is air in the infusion system, and uses the plunger force profile and the pressure profile to determine whether or not an occlusion is present in the infusion system. The method 368 may utilize the system of Figure 1. In other embodiments, the method 368 may utilizing varying systems.

In step 370, the method starts. The method proceeds from step 370 to step 372. In step 372, the variables are set including setting a sampling step k = 1, a baseline which is a force profile associated with fluid, setting a force threshold for air detection Air_Thr, setting a force threshold for occlusion detection Occl_Thr, setting a pressure threshold Press_Thr, and setting a forgetting factor λ. It is noted that throughout this disclosure that k represents one stroke of the pump of the infusion system. The method proceeds from step 372 through location step 374 to step 376. In step 376, a force profile X(k) is acquired for the current sample of the pumping cycle of the infusion system. It is noted that the force profile X(k) represents a plurality of force readings which are taken during each stroke k of the pump. For instance, in one embodiment six force readings may be taken at various points of each stroke k of the pump. In other embodiments, any number of force readings may be taken throughout each stroke k of the pump. The method proceeds from step 376 through location step 378 to step 382.

While the method proceeds from location step 374 to step 376, the method also simultaneously proceeds from location step 374 to step 380. In step 380, a pressure profile Y(k) is acquired for the current sample of the pumping cycle of the infusion system. It is noted that the pressure profile Y(k) represents a plurality of pressure readings which are taken during each stroke k of the pump. For instance, in one embodiment six pressure readings may be taken at various points of each stroke k of the pump. In other embodiments, any number of pressure readings may be taken throughout each stroke k of the pump. The method proceeds from step 380 through location step 378 to step 382.

In step 382, a force difference D(k) for the current sample k of the pumping cycle is determined by subtracting the baseline from the force profile X(k) for the current sample k, wherein the equation is D(k) = X(k) - baseline. The method proceeds from step 382 to step 384. In step 384, a determination is made as to whether the minimum value of the force difference min(D(k)) for the current sample k is less than the force threshold for air detection Air_Thr. If the determination is made in step 384 that the minimum value of the force difference min(D(k)) for the current sample k is less than the force threshold for air detection Air_Thr then the method proceeds to step 386. This drop in the force profile indicates a transition from fluid to air since air is more compressible than fluid resulting in less force. In step 386, a determination is made that air has been detected and a qualifier algorithm may be applied to determine whether to stop the infusion.

If the determination is made in step 384 that the minimum value of the force difference min(D(k)) for the current sample k is not less than the force threshold for air detection Air_Thr then the method proceeds to step 388. In step 388, a determination is made whether the maximum value of the force difference max(D(k)) for the current sample k is greater than or equal to the force threshold for occlusion detection Occl_Thr. If the determination is made in step 388 that the maximum value of the force difference max(D(k)) for the current sample k is not greater than or equal to the force threshold for occlusion detection Occl_Thr then the method proceeds to step 390. It is noted that during an occlusion the plunger force readings are higher than in non-occlusion conditions. In step 390, a determination is made that an occlusion has not been detected and the occlusion indicator OccIndicator is set to 0 because air was not detected and a significant increase in the force difference was not detected. The method proceeds from step 390 to step 392. In step 392, the baseline is updated using the equation baseline = ((1- forgetting factor λ) ^{∗} baseline) + (forgetting factor λ ^{∗} force profile X(k)). It is noted that unless an occlusion is detected, the method updates the baseline to account for the variability seen in the force-profiles due to medication type, tubing type, PMC, ambient temperature, or other factors. The method proceeds from step 392 to step 394. In step 394, the sampling step k is increased using the equation k = k + 1. The method proceeds from step 394 to location step 374 and repeats the process steps.

If the determination is made in step 388 that the maximum value of the force difference max(D(k)) for the current sample k is greater than or equal to the force threshold for occlusion detection Occl_Thr then the method proceeds to step 396. In step 396, a determination is made as to whether the maximum value of the pressure profile max(Y(k)) for the current sample k is greater than the pressure threshold Press_Thr. If the determination is made in step 396 that the maximum value of the pressure profile max(Y(k)) for the current sample k is not greater than the pressure threshold Press_Thr then the method proceeds to step 390. In step 390, a determination is made that an occlusion has not been detected and the occlusion indicator OccIndicator is set to 0 because air was not detected, and although a significant increase in the force difference was detected a significant increase in the pressure profile was not detected. The method proceeds from step 390 to step 392. In step 392, the baseline is updated using the equation baseline = ((1- forgetting factor λ) ^{∗} baseline) + (forgetting factor λ ^{∗} force profile X(k)). It is noted that unless an occlusion is detected, the method updates the baseline to account for the variability seen in the force-profiles due to medication type, tubing type, PMC, ambient temperature, or other factors. The method proceeds from step 392 to step 394. In step 394, the sampling step k is increased using the equation k = k + 1. The method proceeds from step 394 to location step 374 and repeats the process steps.

If the determination is made in step 396 that the maximum value of the pressure profile max(Y(k)) for the current sample k is greater than the pressure threshold Press_Thr then the method proceeds to step 398. In step 398, a determination is made that an occlusion has been detected and the occlusion indicator OccIndicator is set to 1 because air was not detected, a significant increase in the force profile was detected, and a significant increase in the pressure profile was detected. The method proceeds from step 398 to step 400. In step 400, the baseline is not updated so that the baseline = baseline. The baseline is not updated to eliminate/discard the changes in the force measurement which may be caused by the applied pressure/occlusion in order to eliminate false air-detections. The method proceeds from step 400 to step 394. In step 394, the sampling step k is increased using the equation k = k + 1. The method proceeds from step 394 to location step 374 and repeats the process steps. In other embodiments, the method 368 of Figure 12 may be altered to vary the order or substance of any of the steps, to delete one or more steps, or to add one or more steps.

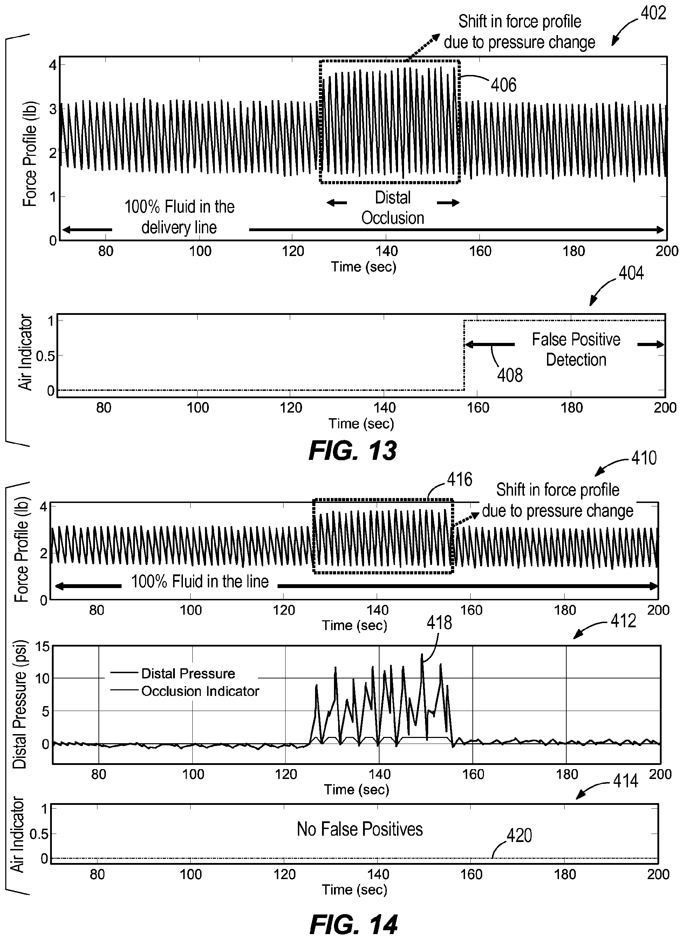

Figure 13 illustrates two related graphs 402 and 404 illustrating how the use of a single-sensor based algorithm for detecting the presence of air within an infusion system can lead to a false positive detection of air in the infusion system. The graphs 402 and 404 were taken from an infusion system which did not contain a significant amount of air to warrant stopping the infusion system but rather underwent a temporary distal occlusion during a portion of the testing. The X-axis of graph 402 represents time in seconds during the infusion of infusion fluid delivered by the infusion system. The Y-axis of graph 402 represents force sensor readings of the single force sensor in pounds for the infusion system during the infusion of the infusion fluid. The portion 406 of the plotted force profile readings shows that the force profile readings substantially increase and then decrease around 125 seconds to 155 seconds when the distal occlusion occurred.

Similarly, the X-axis of graph 404 represents the corresponding time in seconds during the infusion of infusion fluid delivered by the infusion system. The Y-axis of graph 404 represents air indicator determinations made based on the force profile sensor readings of the corresponding graph 402. The portion 408 of the plotted air indicator readings shows that the air indicator readings substantially increase around 155 seconds based on the force profile sensor readings of the corresponding graph 402 which were due to the temporary distal occlusion. This portion 404 would result in a false positive detection of air when using a typical single-sensor based force-profile algorithm. This false positive is caused by the temporary occlusion. This is problematic as the infusion system would be shut down due to this false positive creating an improper delay in therapy to the patient.

Figure 14 illustrates three related graphs 410, 412, and 414 illustrating how the use of a multi-sensor based algorithm for detecting the presence of air within the infusion system tested in Figure 13 eliminates the false positive detection of air in the in the infusion system. The graphs 410, 412, and 414 were taken from the infusion system tested in Figure 13 which did not contain a significant amount of air to warrant stopping the infusion system but which underwent a temporary occlusion during a portion of the testing. The X-axis of graph 410 represents time in seconds during the infusion of infusion fluid delivered by the infusion system. The Y-axis of graph 410 represents force profile sensor readings in pounds for the infusion system during the infusion of the infusion fluid. The portion 416 of the plotted force profile sensor readings shows that the force-sensor readings substantially increase and then decrease around 125 seconds to 155 seconds due to the temporary occlusion.

Similarly, the X-axis of graph 412 represents the corresponding time in seconds during the infusion of infusion fluid delivered by the infusion system. The Y-axis of graph 412 represents distal pressure readings in psi taken by a distal pressure sensor connected to the infusion system. The plotted portion 418 of the distal pressure readings shows that the distal pressure substantially increases and then decreases around 125 seconds to 155 seconds which indicates that an occlusion was present and then dissolved and may have been the reason that the force profile of graph 410 increased from the period of around 125 seconds to 155 seconds. The decrease of the force profile of graph 410 around 155 seconds may have been due to the release of the occlusion rather than air being in the infusion system.

Similarly, the X-axis of graph 414 represents the corresponding time in seconds during the infusion of infusion fluid delivered by the infusion system. The Y-axis of graph 414 represents air indicator determinations made by integrating the force profile readings and the distal pressure readings of the corresponding graphs 410, and 412. The plotted portion 420 of the air indicator readings shows that the air indicator readings stayed at 0 during the entire time plotted from 0 seconds to 200 seconds based on the integrated force profile readings and the distal pressure readings of the corresponding graphs 410 and 412. As a result, the use of multiple different types of sensors to monitor the infusion system has eliminated the false positive detection of air which occurred when the same infusion system was tested using only a single type of sensor when a temporary occlusion was present. This improves accuracy and avoids unnecessary shut-downs of the infusion system.

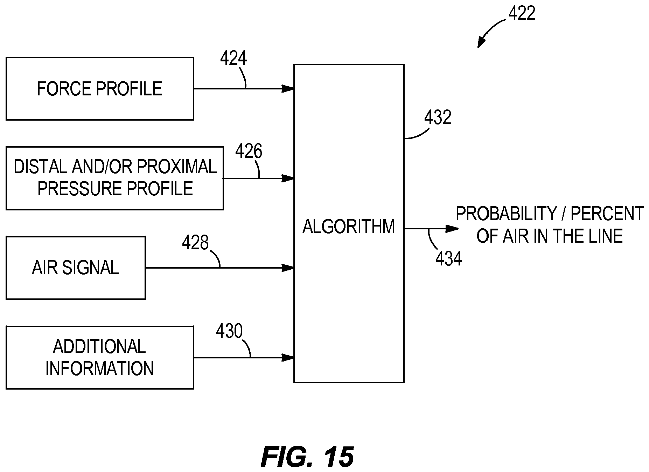

Figure 15 illustrates a flowchart of an embodiment of a method 422 for determining a probability of air being in an infusion system. The method 422 may utilize the system of Figure 1. In other embodiments, the method 422 may utilize varying systems. In step 424, a force sensor determines how much force is acting upon a plunger or pumping member of a pump of the infusion system. In step 426, a distal pressure sensor and/or a proximal pressure sensor determines the distal pressure and/or the proximal pressure acting upon the infusion system. In step 428, an air-in-line sensor determines how much of a signal propagates through a fluid-delivery line of the infusion system. In step 430, additional information is determined. The additional information may comprise medication information regarding the infusion fluid. The medication information may comprise a formulation of the infusion fluid, a rate of the infusion fluid, a duration of the infusion fluid, a viscosity of the infusion fluid, a therapy type of the infusion fluid, or a property of the infusion fluid. The additional information may comprise infusion information regarding the infusion of the infusion fluid. The infusion information may comprise a volume of the infusion fluid in the infusion container or another parameter regarding the infusion. In step 432, the measurements and information from steps 424, 426, 428, and 430 are used in an algorithm to integrate the measurements and information. In step 434, a determination is made as to the probability/percent chance of air being disposed in the fluid delivery line of the infusion system based on the results of the algorithm applied in step 432. In other embodiments, the method 422 of Figure 15 may be altered to vary the order or substance of any of the steps, to delete one or more steps, or to add one or more steps.

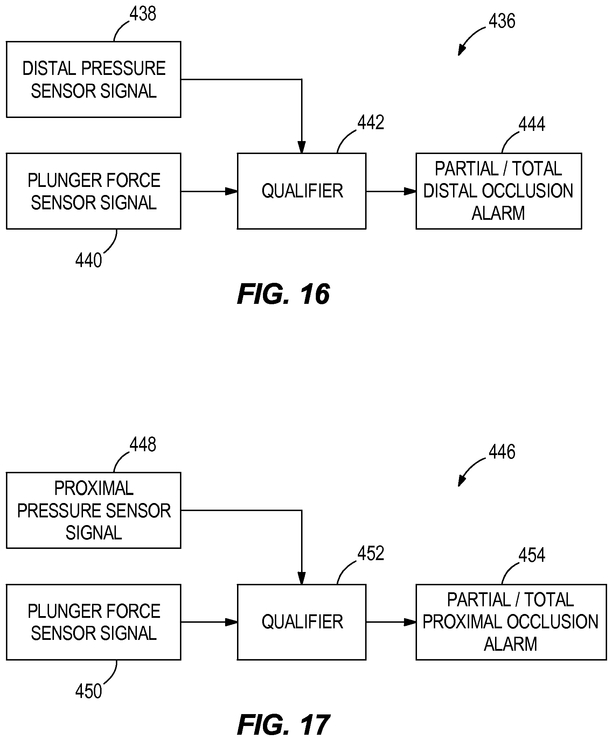

Figure 16 illustrates a flowchart of an embodiment of a method 436 for determining whether a partial or total distal occlusion is present in an infusion system. The method 436 may utilize the system of Figure 1. In other embodiments, the method 436 may utilize varying systems. In step 438, a distal pressure sensor determines the amount of distal pressure acting on the infusion system. In step 440, a plunger force sensor determines how much force is acting upon a plunger or pumping member of a pump of the infusion system. In step 442, a qualifier integrates the results of steps 438 and 440 using one or more algorithms. In step 444, a determination is made as to whether there is a partial or total distal occlusion in the infusion system based on the qualifier used in step 442 and if there is an alarm is turned on allowing the infusion system to be turned off. The use of the results of multiple different types of sensors in one or more algorithms improves the robustness and false positive performance of the occlusion detection system. In other embodiments, the method 436 of Figure 16 may be altered to vary the order or substance of any of the steps, to delete one or more steps, or to add one or more steps.

Figure 17 illustrates a flowchart of another embodiment of a method 446 for determining whether a partial or total proximal occlusion is present in an infusion system. The method 446 may utilize the system of Figure 1. In other embodiments, the method 446 may utilize varying systems. In step 448, a proximal pressure sensor determines the amount of proximal pressure acting on the infusion system. In step 450, a plunger force sensor determines how much force is acting upon a plunger or pumping member of a pump of the infusion system. In step 452, a qualifier integrates the results of steps 448 and 450 using one or more algorithms. In step 454, a determination is made as to whether there is a partial or total proximal occlusion in the infusion system based on the qualifier used in step 452 and if there is an alarm is turned on allowing the infusion system to be turned off. The use of the results of multiple different types of sensors in one or more algorithms improves the robustness and false positive performance of the occlusion detection system. In other embodiments, the method 446 of Figure 17 may be altered to vary the order or substance of any of the steps, to delete one or more steps, or to add one or more steps.

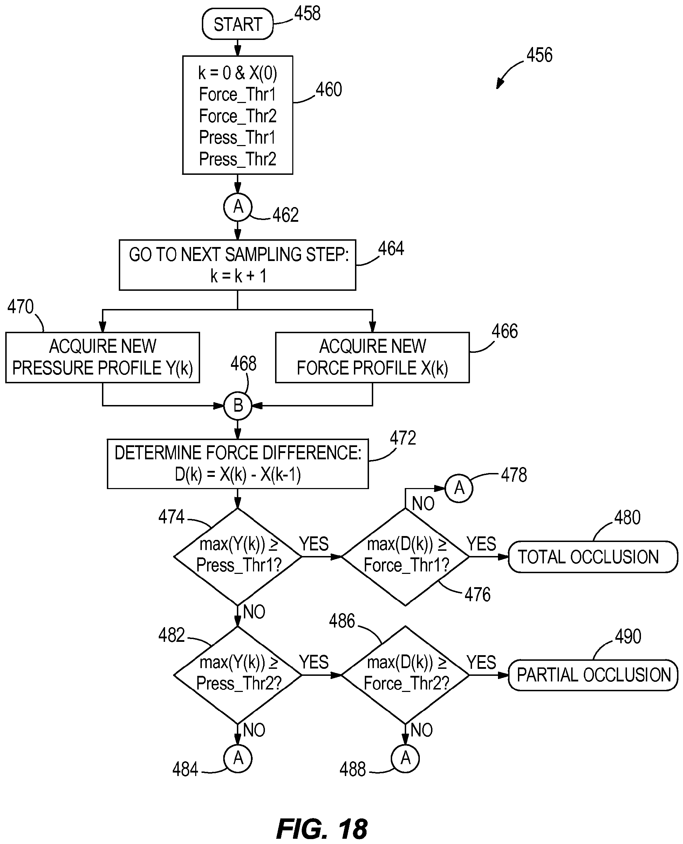

Figure 18 illustrates a flowchart of another embodiment of a method 456 of detecting a partial or total occlusion in an infusion system. It can be applied as long as a plunger force profile and a pressure profile is taken at each sampling step. The method 456 uses the plunger force profile and the pressure profile to determine whether or not a partial or total occlusion is present in the infusion system. The method 456 may utilize the system of Figure 1. In other embodiments, the method 456 may utilizing varying systems.

In step 458, the method starts. The method proceeds from step 458 to step 460. In step 460, the variables are set including sampling step k = 0, setting the initial force profile associated with fluid X(0), setting the force threshold for total occlusion Force_Thr1, setting the force threshold for partial occlusion Force_Thr2, setting the pressure threshold for total occlusion Press_Thr1, and setting the pressure threshold for partial occlusion Press_Thr2. It is noted that the force threshold for total occlusion Force_Thr1 is greater than the force threshold for partial occlusion Force_Thr2. It is further noted that the pressure threshold for total occlusion Press_Thr1 is greater than the pressure threshold for partial occlusion Press_Thr2. The method proceeds from step 460 through location step 462 to step 464. In step 464, the sampling step k is set to k = k + 1. The method proceeds from step 464 to step 466. In step 466, a force sensor is used to determine a force profile X(k) at sampling step k based on measurements of the force sensor. It is noted that the force profile X(k) represents a plurality of force readings which are taken during each stroke k of the pump. For instance, in one embodiment six force readings may be taken at various points of each stroke k of the pump. In other embodiments, any number of force readings may be taken throughout each stroke k of the pump. The method proceeds from step 466 through location step 468 to step 472.

While the method proceeds from step 464 to step 466, the method also simultaneously proceeds from step 464 to step 470. In step 470, a pressure sensor is used to determine a pressure profile Y(k) at sampling step k based on measurements of the pressure sensor. It is noted that the pressure profile Y(k) represents a plurality of pressure readings which are taken during each stroke k of the pump. For instance, in one embodiment six pressure readings may be taken at various points of each stroke k of the pump. In other embodiments, any number of pressure readings may be taken throughout each stroke k of the pump. The method proceeds from step 470 through location step 468 to step 472. In step 472, a force difference D(k) at sampling step k is determined by using the equation force profile X(k) - force profile X(k-1) (i.e. subtracting the force profile for the previous sampling step X(k-1) from the current sampling step force profile X(k)). The method proceeds from step 472 to step 474. In step 474, a determination is made as to whether the maximum pressure profile max(Y(k)) for the current sample k is greater than or equal to the pressure threshold for total occlusion Press_Thr1. If in step 474 a determination is made that the maximum pressure profile max(Y(K)) for the current sample k is greater than or equal to the pressure threshold for total occlusion Press_Thr1 then the method proceeds to step 476. In step 476, a determination is made as to whether the maximum force difference max(D(k)) for the current sample k is greater than or equal to the force threshold for total occlusion Force_Thr1. If step 476 determines that the maximum force difference max(D(k)) for the current sample k is not greater than or equal to the force threshold for total occlusion Force_Thr1 then the method proceeds through location step 478 through location step 462 to step 464 and repeats the process steps.

If step 476 determines that the maximum force difference max(D(k)) for the current sample k is greater than or equal to the force threshold for total occlusion Force_Thr1 then the method proceeds to step 480 and determines that there is a total occlusion. At this point, an alarm may be turned on and/or the infusion system may be turned off.

If in step 474 a determination is made that the maximum pressure profile max(Y(K)) for the current sample k is not greater than or equal to the pressure threshold for total occlusion Press_Thr1 then the method proceeds to step 482. In step 482, a determination is made as to whether the maximum pressure profile max(Y(k)) for the current sample k is greater than or equal to the pressure threshold for partial occlusion Press_Thr2. If step 482 determines that the maximum pressure profile max(Y(k)) for the current sample k is not greater than or equal to the pressure threshold for partial occlusion Press_Thr2 then the method proceeds from step 482 through location step 484 though location step 462 to step 464 and repeats the process steps.

If step 482 determines that the maximum pressure profile max(Y(k)) for the current sample k is greater than or equal to the pressure threshold for partial occlusion Press_Thr2 then the method proceeds from step 482 to step 486. In step 486, a determination is made as to whether the maximum force difference max(D(k)) for the current sample k is greater than or equal to the force threshold for partial occlusion Force_Thr2. If step 486 determines that the maximum force difference max(D(k)) for the current sample k is not greater than or equal to the force threshold for partial occlusion Force_Thr2 then the method proceeds through location step 488 through location step 462 to step 464 and repeats the process steps.

If step 486 determines that the maximum force difference max(D(k)) for the current sample k is greater than or equal to the force threshold for partial occlusion Force_Thr2 then the method proceeds to step 490 and determines that there is a partial occlusion. At this point an alarm may be generated or turned on and/or the infusion system may be turned off.

## Claims

1. An infusion system (100) for being operatively connected to a fluid delivery line (104) and to an infusion container (102) containing an infusion fluid, the infusion system comprising:
a pump (106);
a plurality of different types of sensors (114) connected to the pump or the fluid delivery line, the plurality of different types of sensors configured to indicate whether air is in the fluid delivery line, wherein the plurality of different types of sensors comprise an air sensor and a force sensor;
at least one processor (108) in electronic communication with the pump and the plurality of different types of sensors; and
a memory in electronic communication with the at least one processor, wherein the memory comprises
programming code for execution by the at least one processor, and the programming code is configured to, based on measurements taken by the plurality of different types of sensors, determine the following:
(1) whether there is air in the fluid delivery line;
(2) whether there is a partial occlusion or total occlusion in the fluid delivery line; or
(3) a percentage of the air present in the fluid delivery line or the probability of the air being in the fluid delivery line;
**characterized in that**
the programming code is further configured to adjust a sensitivity of measurements determined from the air sensor based on a measurement determined from the force sensor.

2. The infusion system of claim 1, wherein the plurality of the different types of sensors further comprise a pressure sensor.

3. The infusion system of claim 1, wherein the programming code is configured to determine the percentage of the air present in the fluid delivery line or the probability of the air being in the fluid delivery line.

4. The infusion system of claim 3, wherein the programming code is configured to determine the percentage of the air present in the fluid delivery line or the probability of the air being in the fluid delivery line based additionally on medication information regarding the infusion fluid or on infusion information regarding the infusion of the infusion fluid.

5. The infusion system of claim 4, wherein the programming code is configured to determine the percentage of the air present in the fluid delivery line or the probability of the air being in the fluid delivery line based additionally on the medication information regarding the infusion fluid, the medication information comprising a formulation of the infusion fluid, a rate of the infusion fluid, a duration of the infusion fluid, a viscosity of the infusion fluid, a therapy of the infusion fluid, or a property of the infusion fluid.

6. The infusion system of claim 4, wherein the programming code is configured to determine the percentage of the air present in the fluid delivery line or the probability of the air being in the fluid delivery line based additionally on the infusion information regarding the infusion of the infusion fluid, the infusion information comprising a volume of the infusion fluid in the infusion container or another parameter regarding the infusion.

7. The infusion system of claim 1, wherein the programming code is configured to compensate for at least one distance between the plurality of different types of sensors, which leads to one or more delays in the measurements, by using or comparing the measurements taken by the plurality of the different types of sensors at different portions of cycles of pumping of the pump to accommodate for the one or more delays in the measurements.

8. The infusion system of claim 1, wherein the programming code is further configured to based on the measurements taken by the plurality of the different types of sensors: (1) adjust a sensitivity of at least one of the plurality of different types of sensors; (2) determine a nuisance air alarm; (3) determine a missed air alarm; or (4) update a baseline profile.

9. A method for adjusting a sensitivity of measurements taken by sensors connected to an infusion system, the infusion system comprising a fluid delivery line, the method comprising:
taking measurements with a plurality of different types of sensors connected to the infusion system, wherein the plurality of different types of sensors comprise an air sensor and a force sensor;
determining with at least one processor, based on the measurements taken by the plurality of the different types of the sensors, one of the following:
(1) whether there is air in the fluid delivery line;
(2) whether there is a partial occlusion or a total occlusion in the fluid delivery line; or
(3) a percentage of the air present in the fluid delivery line or the probability of the air being in the fluid delivery line; and
adjusting a sensitivity of measurements determined from the air sensor based on a measurement determined from the force sensor.

10. The method of claim 9, wherein the plurality of different types of sensors further comprise a pressure sensor.

## Patentansprüche

1. Infusionssystem (100) zum funktionsfähigen Verbinden mit einer Fluidförderleitung (104) und mit einem Infusionsbehälter (102), der ein Infusionsfluid aufweist, wobei das Infusionssystem umfasst:
eine Pumpe (106);
eine Vielzahl von verschiedenen Arten von Sensoren (114), die mit der Pumpe oder der Fluidförderleitung verbunden sind, wobei die Vielzahl von verschiedenen Arten von Sensoren konfiguriert ist, um anzuzeigen, ob sich Luft in der Fluidförderleitung befindet, wobei die Vielzahl von verschiedenen Arten von Sensoren einen Luftsensor und einen Kraftsensor umfasst;
mindestens einen Prozessor (108) in elektronischer Verbindung mit der Pumpe und der Vielzahl von verschiedenen Arten von Sensoren; und
einen Speicher in elektronischer Verbindung mit dem mindestens einen Prozessor, wobei der Speicher umfasst
Programmiercode zur Ausführung durch den mindestens einen Prozessor und der Programmiercode ist konfiguriert, um basierend auf Messungen, die von der Vielzahl von verschiedenen Arten von Sensoren durchgeführt werden, das Folgende zu bestimmen:
(1) ob sich Luft in der Fluidförderleitung befindet;
(2) ob es einen teilweisen Verschluss oder einen vollständigen Verschluss in der Fluidförderleitung gibt; oder
(3) einen Prozentsatz der in der Fluidförderleitung vorhandenen Luft oder die Wahrscheinlichkeit, dass sich die Luft in der Fluidförderleitung befindet;
**dadurch gekennzeichnet, dass**
der Programmiercode weiter konfiguriert ist, um eine Empfindlichkeit von Messungen, die von dem Luftsensor bestimmt werden, basierend auf einer von dem Kraftsensor bestimmten Messung einzustellen.

2. Infusionssystem nach Anspruch 1, wobei die Vielzahl von den verschiedenen Arten von Sensoren weiter einen Drucksensor umfasst.

3. Infusionssystem nach Anspruch 1, wobei der Programmiercode konfiguriert ist, um den Prozentsatz der in der Fluidförderleitung vorhandenen Luft oder die Wahrscheinlichkeit, dass sich die Luft in der Fluidförderleitung befindet, zu bestimmen.

4. Infusionssystem nach Anspruch 3, wobei der Programmiercode konfiguriert ist, um den Prozentsatz der in der Fluidförderleitung vorhandenen Luft oder die Wahrscheinlichkeit, dass sich die Luft in der Fluidförderleitung befindet, basierend zusätzlich auf Medikamenteninformationen bezüglich des Infusionsfluides oder auf Infusionsinformation bezüglich der Infusion des Infusionsfluides zu bestimmen.

5. Infusionssystem nach Anspruch 4, wobei der Programmiercode konfiguriert ist, um den Prozentsatz der in der Fluidförderleitung vorhandenen Luft oder die Wahrscheinlichkeit, dass sich die Luft in der Fluidförderleitung befindet, basierend zusätzlich auf den Medikamenteninformationen bezüglich des Infusionsfluides zu bestimmen, wobei die Medikamenteninformationen eine Formulierung des Infusionsfluides, eine Rate des Infusionsfluides, eine Dauer des Infusionsfluides, eine Viskosität des Infusionsfluides, eine Therapie des Infusionsfluides oder eine Eigenschaft des Infusionsfluides umfassen.

6. Infusionssystem nach Anspruch 4, wobei der Programmiercode konfiguriert ist, um den Prozentsatz der in der Fluidförderleitung vorhandenen Luft oder die Wahrscheinlichkeit, dass sich die Luft in der Fluidförderleitung befindet, basierend zusätzlich auf den Infusionsinformationen bezüglich der Infusion des Infusions-fluides zu bestimmen, wobei die Infusionsinformationen ein Volumen des Infusionsfluides in dem Infusionsbehälter oder einen anderen Parameter bezüglich der Infusion umfassen.

7. Infusionssystem nach Anspruch 1, wobei der Programmiercode konfiguriert ist, um mindestens einen Abstand zwischen der Vielzahl von verschiedenen Arten von Sensoren zu kompensieren, der zu einer oder mehreren Verzögerungen in den Messungen führt, durch Verwenden oder Vergleichen der von der Vielzahl der verschiedenen Arten von Sensoren an verschiedenen Abschnitten von Pump-zyklen der Pumpe durchgeführten Messungen, um die eine oder mehreren Verzögerungen in den Messungen auszugleichen.

8. Infusionssystem nach Anspruch 1, wobei der Programmiercode weiter konfiguriert ist, um basierend auf den von der Vielzahl der verschiedenen Arten von Sensoren durchgeführten Messungen: (1) eine Empfindlichkeit von mindestens einem der Vielzahl von verschiedenen Arten von Sensoren einzustellen; (2) einen störende Luft-Alarm zu bestimmen; (3) einen fehlende Luft-Alarm zu bestimmen; oder (4) ein Basisprofil zu aktualisieren.

9. Verfahren zum Einstellen einer Empfindlichkeit von Messungen, die von Sensoren durchgeführt werden, die mit einem Infusionssystem verbunden sind, wobei das Infusionssystem eine Fluidförderleitung umfasst, wobei das Verfahren umfasst:
Durchführen von Messungen mit einer Vielzahl von verschiedenen Arten von Sensoren, die mit dem Infusionssystem verbunden sind, wobei die Vielzahl von verschiedenen Arten von Sensoren einen Luftsensor und einen Kraftsensor umfasst;
Bestimmen mit mindestens einem Prozessor basierend auf den Messungen, die von der Vielzahl der verschiedenen Arten von Sensoren durchgeführt werden, eines der Folgenden:
(1) ob sich Luft in der Fluidförderleitung befindet;
(2) ob es einen teilweisen Verschluss oder einen vollständigen Verschluss in der Fluidförderleitung gibt; oder
(3) einen Prozentsatz der in der Fluidförderleitung vorhandenen Luft oder die Wahrscheinlichkeit, dass sich die Luft in der Fluidförderleitung befindet; und
Einstellen der Empfindlichkeit von Messungen, die von dem Luftsensor bestimmt werden, basierend auf einer Messung, die von dem Kraftsensor bestimmt wird.

10. Verfahren nach Anspruch 9, wobei die Vielzahl von verschiedenen Arten von Sensoren weiter einen Drucksensor umfasst.

## Revendications

1. Système de perfusion (100) pour être connecté fonctionnellement à une conduite de distribution de fluide (104) et à un récipient de perfusion (102) contenant un fluide de perfusion, le système de perfusion comprenant :
une pompe (106) ;
une pluralité de différents types de capteurs (114) connectés à la pompe ou à la conduite de distribution de fluide, la pluralité de différents types de capteurs étant configurés pour indiquer si de l'air se trouve dans la conduite de distribution de fluide, dans lequel la pluralité de différents types de capteurs comprennent un capteur d'air et un capteur de force ;
au moins un processeur (108) en communication électronique avec la pompe et la pluralité de différents types de capteurs ; et
une mémoire en communication électronique avec l'au moins un processeur, dans lequel la mémoire comprend
un code de programmation pour exécution par l'au moins un processeur, et le code de programmation est configuré pour, sur la base de mesures prises par la pluralité de différents types de capteurs, déterminer ce qui suit :
(1) s'il y a de l'air dans la conduite de distribution de fluide ;
(2) s'il y a une occlusion partielle ou une occlusion totale dans la conduite de distribution de fluide ; ou
(3) un pourcentage de l'air présent dans la conduite de distribution de fluide ou la probabilité pour que l'air se trouve dans la conduite de distribution de fluide ;
**caractérisé en ce que**
le code de programmation est en outre configuré pour régler une sensibilité de mesures déterminées à partir du capteur d'air sur la base d'une mesure déterminée à partir du capteur de force.

2. Système de perfusion selon la revendication 1, dans lequel la pluralité de différents types de capteurs comprennent en outre un capteur de pression.

3. Système de perfusion selon la revendication 1, dans lequel le code de programmation est configuré pour déterminer le pourcentage de l'air présent dans la conduite de distribution de fluide ou la probabilité pour que l'air se trouve dans la conduite de distribution de fluide.

4. Système de perfusion selon la revendication 3, dans lequel le code de programmation est configuré pour déterminer le pourcentage de l'air présent dans la conduite de distribution de fluide ou la probabilité pour que l'air se trouve dans la conduite de distribution de fluide sur la base en outre des informations de médicament concernant le fluide de perfusion ou des informations de perfusion concernant la perfusion du fluide de perfusion.

5. Système de perfusion selon la revendication 4, dans lequel le code de programmation est configuré pour déterminer le pourcentage de l'air présent dans la conduite de distribution de fluide ou la probabilité pour que l'air se trouve dans la conduite de distribution de fluide sur la base en outre des informations de médicament concernant le fluide de perfusion, les informations de médicament comprenant une formulation du fluide de perfusion, un débit du fluide de perfusion, une durée du fluide de perfusion, une viscosité du fluide de perfusion, une thérapie du fluide de perfusion, ou une propriété du fluide de perfusion.

6. Système de perfusion selon la revendication 4, dans lequel le code de programmation est configuré pour déterminer le pourcentage de l'air présent dans la conduite de distribution de fluide ou la probabilité pour que l'air se trouve dans la conduite de distribution de fluide sur la base en outre des informations de perfusion concernant la perfusion du fluide de perfusion, les informations de perfusion comprenant un volume du fluide de perfusion dans le récipient de perfusion ou un autre paramètre concernant la perfusion.

7. Système de perfusion selon la revendication 1, dans lequel le code de programmation est configuré pour compenser au moins une distance entre la pluralité de différents types de capteurs, ce qui conduit à un ou plusieurs retards dans les mesures, en utilisant ou en comparant les mesures prises par la pluralité de différents types de capteurs à différentes portions de cycles de pompage de la pompe pour s'adapter aux un ou plusieurs retards dans les mesures.

8. Système de perfusion selon la revendication 1, dans lequel le code de programmation est en outre configuré pour, sur la base des mesures prises par la pluralité de différents types de capteurs : (1) régler une sensibilité d'au moins l'un de la pluralité de différents types de capteurs ; (2) déterminer une alarme d'air nuisible ; (3) déterminer une alarme d'air manqué; ou (4) mettre à jour un profil de base de référence.

9. Procédé pour régler une sensibilité de mesures prises par des capteurs connectés à un système de perfusion, le système de perfusion comprenant une conduite de distribution de fluide, le procédé comprenant :
la prise de mesures avec une pluralité de différents types de capteurs connectés au système de perfusion, dans lequel la pluralité de différents types de capteurs comprennent un capteur d'air et un capteur de force ;
la détermination avec au moins un processeur, sur la base des mesures prises par la pluralité de différents types de capteurs, l'un des éléments suivants :
(1) s'il y a de l'air dans la conduite de distribution de fluide ;
(2) s'il y a une occlusion partielle ou une occlusion totale dans la conduite de distribution de fluide ; ou
(3) un pourcentage de l'air présent dans la conduite de distribution de fluide ou la probabilité pour que l'air se trouve dans la conduite de distribution de fluide ; et
le réglage d'une sensibilité des mesures déterminées à partir du capteur d'air sur la base d'une mesure déterminée à partir du capteur de force.

10. Procédé selon la revendication 9, dans lequel la pluralité de différents types de capteurs comprennent en outre un capteur de pression.
